# EUROPÄISCHE PATENTSCHRIFT

(11) **EP 1 827 267 B1**
(45) Veröffentlichungstag und Bekanntmachung des Hinweises auf die Patenterteilung: **14.10.2015**
(21) Anmeldenummer: 05803589.0
(22) Anmeldetag: 26.10.2005
(51) Int. Cl.: A61B 17/70

(54) **ORTHOPÄDISCHE FIXATIONSVORRICHTUNG UND ORTHOPÄDISCHES FIXATIONSSYSTEM**
ORTHOPEDIC FIXATION DEVICE AND ORTHOPEDIC FIXATION SYSTEM
DISPOSITIF DE FIXATION ORTHOPEDIQUE, ET SYSTEME DE FIXATION ORTHOPEDIQUE

(30) Priorität: 12.11.2004 DE 102004056091
(43) Veröffentlichungstag der Anmeldung: 05.09.2007
(73) Patentinhaber: Aesculap AG, 78532 Tuttlingen (DE)
(72) Erfinder: SCHUMACHER, Jörg, 78532 Tuttlingen (DE); KRAMER, Ulrich, 8374 Oberwangen (CH); BEGER, Jens, 78532 Tuttlingen (DE); LINDNER, Stephan, 78532 Tuttlingen (DE)
(74) Vertreter: Hoeger, Stellrecht & Partner Patentanwälte mbB
(86) Internationale Anmeldenummer: PCT/EP2005/011455
(87) Internationale Veröffentlichungsnummer: WO 2006/050815

(56) Entgegenhaltungen:
- EP-A- 0 524 441
- WO-A-01/52758
- WO-A-01/78613
- WO-A-2004/021901
- US-A- 5 611 800
- US-A- 6 001 098
- US-A1- 2002 143 332

## Beschreibung

Die Erfindung betrifft eine orthopädische Fixationsvorrichtung zum Verbinden eines in oder an einem Knochen verankerbaren, einen Verbindungsabschnitt aufweisenden ersten Verankerungselements mit einem Verbindungselement, welches mit einem zweiten, in oder an einem Knochen verankerbaren Verankerungselement verbindbar ist, wobei die Fixationsvorrichtung in einer Justierstellung relativ zum Verbindungselement und zum Verankerungselement bewegbar ist und in einer Fixierstellung am Verbindungselement und am Verankerungselement festlegbar ist, wobei ein einziges beweglich gelagertes Spannglied vorgesehen ist zum Überführen der Fixationsvorrichtung von der Justierstellung in die Fixierstellung und umgekehrt.

Ferner betrifft die Erfindung ein orthopädisches Fixationssystem umfassend mindestens zwei in oder an einem Knochen verankerbare Verankerungselemente, mindestens ein Verbindungselement zum Verbinden der mindestens zwei Verankerungselemente und mindestens eine orthopädische Fixationsvorrichtung zum Verbinden des mindestens einen Verbindungselements mit einem Verbindungsabschnitt eines der mindestens zwei Verankerungselemente.

Orthopädische Fixationsvorrichtungen und Fixationssysteme, wie sie eingangs beschrieben sind, werden verwendet, um unterschiedliche Knochen oder Knochenfragmente relativ zueinander zu positionieren und zu fixieren. Dazu werden üblicherweise Verankerungselemente in Form von Knochenschrauben oder Knochenhaken in die jeweiligen Knochenfragmente eingesetzt und mittels eines Verbindungselements, welches an beiden Verankerungselementen festgelegt wird, dauerhaft oder temporär verbunden. Bekannt sind Fixationsvorrichtungen, mit denen das Verbindungselement seitlich versetzt zum Verbindungsabschnitt am Verankerungselement festgelegt werden kann. Die Verbindung zwischen Verankerungselement und Verbindungselement kann nach Einsetzen des Verankerungselements hergestellt werden, sodaß das Verankerungselement selbst, beispielsweise eine Knochenschraube, nicht mehr im Knochen verdreht werden muß, was zu einer unerwünschten Lockerung einer einmal eingedrehten Schraube führen kann.

Im Gegensatz zu Knochenschrauben, welche einen Gabelkopf zum Aufnehmen eines Verbindungselements aufweisen, erweist sich das Herstellen einer Verbindung zwischen dem Verankerungselement und dem Verbindungselement bei bekannten Fixationsvorrichtungen als äußerst aufwendig, da zum einen die Fixationsvorrichtung am Verankerungselement festgelegt und zum anderen das Verbindungselement an der Fixationsvorrichtung festgelegt werden muß.

Eine Fixationsvorrichtung der eingangs beschriebenen Art ist beispielsweise aus der WO 2004/021901 A1 bekannt. Weitere Fixationsvorrichtungen sind in der US 2002/0143332 A1, in der US 5,611,800 sowie in der WO 01/52758 A1 offenbart.

Es ist daher Aufgabe der vorliegenden Erfindung, eine orthopädische Fixationsvorrichtung sowie ein orthopädisches Fixationssystem der jeweils eingangs beschriebenen Art so zu verbessern, daß das Verbindungselement am Verankerungselement besonders einfach festlegbar ist.

Diese Aufgabe wird bei einer orthopädischen Fixationsvorrichtung der eingangs beschriebenen Art erfindungsgemäß dadurch gelöst, daß daß eine erste Klemmvorrichtung vorgesehen ist zum klemmenden Festlegen der Fixationsvorrichtung am Verbindungselement in der Fixierstellung, daß eine zweite Klemmvorrichtung vorgesehen ist zum klemmenden Festlegen der Fixationsvorrichtung am Verankerungselement in der Fixierstellung und daß die erste Klemmvorrichtung das Spannglied umfaßt und daß die zweite Klemmvorrichtung das Spannglied umfaßt.

Anders als bei den aus dem Stand der Technik bekannten Fixationsvorrichtungen genügt es, lediglich das Spannglied zu bewegen, wodurch einerseits sowohl die Fixationsvorrichtung am Verbindungselement und andererseits am Verankerungselement festgelegt werden kann. Es ist daher nur ein Verriegelungsschritt notwendig, um gleichzeitig drei Elemente relativ zueinander festzulegen. Dies ist insbesondere vorteilhaft, wenn das Verbindungselement seitlich versetzt relativ zu einer Längsachse des Verankerungselements an diesem festgelegt werden soll. In jedem Fall gestattet es die erfindungsgemäße Fixationsvorrichtung, zunächst das Verankerungselement in gewünschter Weise in einen Knochen oder ein Knochenfragment einzusetzen, danach in gewünschter Weise das Verbindungselement relativ zum Verankerungselement zu justieren und dann in einem einzigen Schritt durch Bewegen des Spannglieds die Fixationsvorrichtung sowohl am Verankerungselement als auch am Verbindungselement festzulegen. Grundsätzlich wäre es denkbar, das Verankerungselement, die Fixationsvorrichtung und das Verbindungselement in der Fixierstellung mittels Formschlußverbindungen festzulegen. Gemäß der Erfindung ist eine erste Klemmvorrichtung vorgesehen zum klemmenden Festlegen der Fixationsvorrichtung am Verbindungselement in der Fixierstellung, eine zweite Klemmvorrichtung zum klemmenden Festlegen der Fixationsvorrichtung am Verankerungselement in der Fixierstellung und daß die erste Klemmvorrichtung das Spannglied umfaßt und daß die zweite Klemmvorrichtung das Spannglied umfaßt. Das Spannglied ist bei der erfindungsgemäßen Ausgestaltung somit Teil beider Klemmvorrichtungen, was es ermöglicht, durch eine Bewegung des Spannglieds gleichzeitig beide Klemmvorrichtungen zu betätigen, das heißt die Fixationsvorrichtung in die Fixierstellung überzuführen, oder zu lösen, das heißt die Fixationsvorrichtung von der Fixierstellung in die Justierstellung zu überführen.

Vorteilhaft ist es, wenn die Fixationsvorrichtung mindestens ein erstes Trägerelement umfaßt und wenn das Spannglied rotierbar an dem mindestens einen ersten Trägerelement gelagert ist. Die Fixationsvorrichtung kann dann von der Justierstellung in die Fixierstellung überführt werden durch einfaches Verdrehen des Spannglieds relativ zum ersten Trägerelement.

Günstig ist es, wenn die Fixationsvorrichtung mindestens ein erstes Trägerelement umfaßt und wenn das Spannglied relativ zum ersten Trägerelement translatierbar gelagert ist. Diese Ausgestaltung, auch in Kombination mit einer möglichen Verdrehbarkeit des Spannglieds relativ zum ersten Trägerelement, gestattet ein Überführen der Fixationsvorrichtung von der Justierstellung in die Fixierstellung durch eine einfache translatorische Bewegung des Spannglieds relativ zum ersten Trägerelement.

Gemäß einer bevorzugten Ausführungsform der Erfindung kann vorgesehen sein, daß das Verbindungselement in der Justierstellung relativ zum Verankerungselement parallel zu sich selbst auf das Verankerungselement hin oder von diesem weg verschiebbar ist. Diese Ausgestaltung ermöglicht es beispielsweise, das Verbindungselement mittels der Fixationsvorrichtung in der Justierstellung am Verankerungselement zu halten und bei Bedarf einen Abstand zwischen dem Verbindungselement und dem Verbindungsabschnitt des Verankerungselements in gewünschter Weise zu verringern, ohne daß das Verbindungselement relativ zum Verankerungselement verschwenkt werden muß.

Um einen besonders guten Halt des Verbindungselements an der Fixationsvorrichtung in der Fixierstellung zu gewährleisten, ist es vorteilhaft, wenn die Fixationsvorrichtung eine zum Verbindungselement korrespondierende Verbindungselementaufnahme zum Aufnehmen des Verbindungselements aufweist. In ähnlicher Weise kann ein optimaler Halt der Fixationsvorrichtung am Verbindungsabschnitt des Verankerungselements sichergestellt werden, wenn die Fixationsvorrichtung eine zum Verbindungsabschnitt korrespondierende Verbindungsabschnittaufnahme zum Aufnehmen des Verbindungsabschnitts aufweist.

Um eine Verschwenkung des Verbindungselements relativ zum Verankerungselement zu ermöglichen, ist es vorteilhaft, wenn die Verbindungsabschnittaufnahme relativ zur Verbindungselementaufnahme um eine erste Drehachse verdrehbar ist.

Vorteilhaft ist es, wenn die Fixationsvorrichtung ein die Verbindungselementaufnahme tragendes erstes Trägerelement und ein am ersten Trägerelement in der Justierstellung um eine Drehachse drehbar gelagertes zweites, die Verbindungsabschnittaufnahme tragendes Trägerelement umfaßt. Die beiden Trägerelemente bilden quasi einen zweiteiligen beweglichen Rahmen und ermöglichen es, das Verbindungselement relativ zum Verankerungselement um die erste Drehachse zu verschwenken.

Besonders einfach wird der Aufbau der Fixationsvorrichtung, wenn das Spannglied eine die erste Drehachse definierende Lagerwelle bildet. Das Spannglied hat somit eine Doppelfunktion, einerseits dient es als Lagerwelle, andererseits als Mittel zum Überführen der Fixationsvorrichtung von der Justierstellung in die Fixierstellung und umgekehrt. Durch diese Konstruktion läßt sich auch der Aufbau der Fixationsvorrichtung vereinfachen.

Vorteilhaft ist es, wenn die erste Klemmvorrichtung ein erstes Klemmelement umfaßt, welches eine an einer ersten Betätigungsfläche eines ersten Klemmstücks des ersten Trägerelement direkt oder indirekt anliegende Aufgleitfläche aufweist, und wenn infolge einer Bewegung des Spannglieds das erste Klemmelement derart bewegbar ist, daß die erste Aufgleitfläche an der ersten Betätigungsfläche aufgleiten und einen Querschnitt der Verbindungselementaufnahme derart verringern kann, sodaß das Verbindungselement in der Verbindungselementaufnahme festlegbar ist. Es ist also möglich, das Spannglied zu bewegen und durch dessen Bewegung quasi das erste Klemmelement anzutreiben, welches direkt oder indirekt ein Klemmstück bewegen kann durch Aufgleiten der Aufgleitfläche an der Betätigungsfläche. Im Ergebnis wird die Fixationsvorrichtung von der Justierstellung in die Fixierstellung überführt, indem der Querschnitt der Verbindungsaufnahme durch die Aufgleitbewegung verringert wird, was zu einem gewünschten Verklemmen des Verbindungselements in der Verbindungselementaufnahme führt.

Um einzelne Teile der Fixationsvorrichtung für unterschiedliche Größen von Trägerelementen verwenden zu können, ist es günstig, wenn ein erstes, zwischen der ersten Aufgleitfläche und der Betätigungsfläche angeordnetes Kraftübertragungsglied vorgesehen ist zum Übertragen einer Kraft vom ersten Klemmelement auf das erste Klemmstück. Beispielsweise könnten die Teile so auch aus unterschiedlichen Materialien gefertigt sein.

Vorteilhaft ist es, wenn die erste Aufgleitfläche und die erste Betätigungsfläche relativ zur ersten Drehachse geneigt sind. Dadurch kann eine Aufgleitbewegung erfolgen, wenn das Spannglied parallel zur Drehachse bewegt wird. Eine parallele Bewegung ergibt sich indirekt auch durch ein Verdrehen des Spannglieds, wenn dieses beispielsweise in Form eines Schraubbolzens ausgebildet ist, der also eine überlagerte Translations-Rotationsbewegung ermöglicht.

Gemäß einer weiteren bevorzugten Ausführungsform der Erfindung kann vorgesehen sein, daß die erste Aufgleitfläche eine erste Außenfläche eines um die erste Drehachse verdrehbaren ersten Exzenters ist und daß die erste Betätigungsfläche mindestens abschnittsweise konkav in Richtung auf die erste Drehachse hin gekrümmt ist. Durch den Exzenter kann infolge einer Verdrehung des Spannglieds ein Abstand zwischen der ersten Außenfläche und der ersten Betätigungsfläche verändert und auf diese Weise eine Klemmung bewirkt werden. Die Betätigungsfläche kann zum Beispiel abschnittsweise hohlzylindrisch geformt sein, die Außenfläche des Exzenters zylindrisch.

Vorteilhafterweise kann das erste Klemmelement in Form eines Konus ausgebildet sein. So kann der Konus eine konische Außenfläche aufweisen, die gleichzeitig als Aufgleitfläche dient. Mit dem Konus kann eine Aufgleitbewegung relativ zur ersten Betätigungsfläche in jeder Drehstellung des Spannglieds erreicht werden, beispielsweise durch eine Translationsbewegung des Konus parallel zur ersten Drehachse.

Ein Zusammenbau der Fixationsvorrichtung wird vereinfacht, wenn das erste Klemmelement und das Spannglied einstückig ausgebildet sind. Auf diese Weise kann auch verhindert werden, daß sich das Klemmelement und das Spannglied in unerwünschter Weise voneinander lösen können.

Je nach Aufbau der Fixationsvorrichtung kann es zum Zusammenbau jedoch vorteilhaft sein, wenn das erste Klemmelement und das Spannglied zweiteilig ausgebildet sind. Dies gestattet es beispielsweise, Teile zuerst zusammenzustecken und dann miteinander fest zu verbinden.

Vorzugsweise sind das erste Klemmelement und das Spannglied drehfest miteinander verbunden. Dies verhindert ein Lösen des ersten Klemmelements vom Spannglied infolge einer Rotation des Spannglieds.

Günstigerweise kann vorgesehen sein, daß das Spannglied einen ersten Außengewindeabschnitt aufweist, daß das erste Klemmelement einen zum ersten Außengewindeabschnitt korrespondierenden ersten Innengewindeabschnitt aufweist und daß durch Drehen des Spannglieds um seine Längsachse das erste Klemmelement parallel zu sich selbst verschiebbar ist. Dieser Aufbau ähnelt dem Aufbau einer Gewindespindel, wobei das Spannglied als Antriebselement eingesetzt wird, um das Klemmelement parallel zu sich selbst verschieben zu können. Vorteilhafterweise ist das Klemmelement hierzu in einer entsprechenden Führung geführt, die eine Rotation des Klemmelements infolge einer Drehbewegung des Spannglieds verhindert.

Günstig kann es ferner sein, wenn die zweite Klemmvorrichtung ein zweites Klemmelement umfaßt, welches eine an einer zweiten Betätigungsfläche des zweiten Trägerelements direkt oder indirekt anliegende zweite Aufgleitfläche aufweist, und wenn infolge einer Bewegung des Spannglieds das zweite Klemmelement derart bewegbar ist, daß die zweite Aufgleitfläche an der zweiten Betätigungsfläche aufgleiten und ein die Verbindungsabschnittaufnahme abschnittsweise begrenzendes Klemmstück derart bewegbar ist, daß sich ein Querschnitt der Verbindungsabschnittaufnahme verringern kann, sodaß der Verbindungsabschnitt des Verankerungselements in der Verbindungsabschnittaufnahme festlegbar ist. Mit dem Spannglied kann das zweite Klemmelement bewegt werden, wodurch eine Aufgleitbewegung zwischen dem Klemmelement und dem direkt oder indirekt an diesem anliegenden Klemmstück ermöglicht wird zum Verringern eines Querschnitts der Verbindungsabschnittaufnahme. Durch die Querschnittsverringerung kann der Verbindungsabschnitt in der Verbindungsabschnittaufnahme festgelegt werden.

Eine Aufgleitbewegung ist auf einfache Weise möglich, wenn die zweite Aufgleitfläche und die zweite Betätigungsfläche relativ zur ersten Drehachse geneigt sind. Ein Aufgleiten wird so ermöglicht, wenn das Spannglied beispielsweise durch eine Bewegung parallel zur ersten Drehachse bewegt wird, sei es durch eine reine Translationsbewegung oder durch eine überlagerte Translations-Rotationsbewegung.

Günstig ist es, wenn die zweite Aufgleitfläche eine zweite Außenfläche eines um die erste Drehachse verdrehbaren zweiten Exzenters ist und wenn die zweite Betätigungsfläche mindestens abschnittsweise konkav in Richtung auf die erste Drehachse hin gekrümmt ist. Der Exzenter, dessen Außenfläche exzentrisch zur ersten Drehachse angeordnet ist, kann einen Abstand zwischen der zweiten Aufgleitfläche und der zweiten Betätigungsfläche verringern, wenn das Spannglied und damit der Exzenter um die erste Drehachse gedreht wird. Die zweite Außenfläche kann eine Zylinderoberfläche sein, die zweite Betätigungsfläche eine hohlzylindrische Fläche oder zumindest ein derartiger Flächenabschnitt.

Ein besonders einfacher Aufbau der Fixationsvorrichtung ergibt sich, wenn das zweite Klemmelement mindestens teilweise in Form eines Konus ausgebildet ist. Dies bedeutet, daß auch nur ein Teil oder ein Abschnitt des zweiten Klemmelements eine konische oder zumindest geneigte Fläche aufweisen können. Ein solches Klemmelement ist besonders einfach herzustellen.

Um die Stabilität der Fixationsvorrichtung zu erhöhen, kann es vorteilhaft sein, wenn das zweite Klemmelement und das Spannelement einstückig ausgebildet sind. Ferner wird so vermieden, daß sich das zweite Klemmelement und das Spannglied voneinander lösen können, wenn dies nicht erwünscht ist.

Vorzugsweise sind das zweite Klemmelement und das Spannglied zweiteilig ausgebildet. Dies kann einen Zusammenbau unterschiedlicher Teile überhaupt erst ermöglichen. Ferner ist beim Zusammenbauen die Kombination unterschiedlicher Spannglieder mit unterschiedlichen Klemmelementen möglich.

Um sicherzustellen, daß infolge einer Drehung des Spannglieds das zweite Klemmelement mit verdreht wird, ist es günstig, wenn das zweite Klemmelement und das Spannglied drehfest miteinander verbunden sind.

Gemäß einer bevorzugten Ausführungsform der Erfindung kann vorgesehen sein, daß das Spannglied einen zweiten Außengewindeabschnitt aufweist, daß das zweite Klemmelement einen zum zweiten Außengewindeabschnitt korrespondierenden zweiten Innengewindeabschnitt aufweist und daß durch Drehen des Spannglieds um seine Längsachse das zweite Klemmelement parallel zu sich selbst verschiebbar ist. Durch diese Ausgestaltung wird quasi ein Spindelantrieb zum Antreiben des zweiten Klemmelements infolge einer Rotation des Spannglieds ausgebildet. Damit sich das zweite Klemmelement infolge einer Drehung des Spannglieds nicht mitdreht, kann eine entsprechende Führung für das zweite Klemmelement vorgesehen sein.

Günstigerweise sind der erste und der zweite Außengewindeabschnitt gegenläufige Gewindeabschnitte. Dies ermöglicht es, daß infolge einer Rotation des Spannglieds die beiden Klemmelemente gleichzeitig aufeinander zu oder voneinander weg bewegt werden können.

Dabei kann es vorteilhaft sein, wenn der erste Außengewindeabschnitt ein Rechtsgewindeabschnitt und wenn der zweite Außengewindeabschnitt ein Linksgewindeabschnitt ist. Dies ermöglicht es, beispielsweise durch eine Rotation des Spannglieds im Uhrzeigersinn, die beiden Klemmstücke mit dem Spannglied derart anzutreiben, daß sie aufeinander zu bewegt werden, wodurch die Fixationsvorrichtung von der Justierstellung in die Fixierstellung überführt werden kann.

Vorteilhaft ist es, wenn sich in der Fixierstellung das zweite Trägerelement über das zweite Klemmstück an dem Verbindungsabschnitt abstützt. Auf diese Weise können Verbindungsabschnitte mit unterschiedlichem Querschnitt von der Verbindungsabschnittaufnahme aufgenommen und in dieser festgelegt werden.

Um möglichst wenig Einzelteile bei der Herstellung der Fixationsvorrichtung zusammenfügen zu müssen, ist es vorteilhaft, wenn das zweite Klemmstück und das zweite Trägerelement einstückig ausgebildet sind. Beispielsweise kann das zweite Klemmstück in Form eines am Trägerelement gehaltenen beweglichen Lappens ausgebildet sein.

Um die Fixationsvorrichtung eventuell während eines chirurgischen Eingriffes an bestimmte Verankerungselemente anpassen zu können, ist es günstig, wenn das zweite Klemmstück und das zweite Trägerelement zweiteilig ausgebildet sind. Dies gestattet es, Klemmstücke gewünschter Stärke oder Dicke auszuwählen und an der Fixationsvorrichtung anzuordnen.

Damit das Verbindungselement parallel zu sich selbst in Richtung auf das Verankerungselement hin bewegt werden kann, ist es vorteilhaft, wenn das zweite Trägerelement in der Justierstellung um eine zweite Drehachse drehbar am Verbindungsabschnitt gelagert ist. Mit anderen Worten bedeutet dies, daß der Verbindungsabschnitt selbst oder das zweite Trägerelement jeweils ein Lagerelement für das jeweils andere Element bilden zur Ausbildung eines Drehlagers.

Ein besonders einfacher Aufbau der Fixationsvorrichtung ergibt sich, wenn die erste und die zweite Drehachse parallel zueinander verlaufen. Zudem wird so eine Bewegung des Verbindungselements parallel zu sich selbst auf das Verankerungselement hin gestattet.

Um einen Neigungswinkel des Verbindungselements relativ zum Verankerungselement einstellen zu können, ist es vorteilhaft, wenn die Verbindungselementaufnahme am ersten Trägerelement um eine dritte Drehachse verdrehbar gelagert ist und wenn die dritte Drehachse quer zu einer Längsachse der Verbindungselementaufnahme verläuft. Die Verdrehbarkeit kann zudem durch entsprechend vorgesehene Winkelanschläge begrenzt sein, so daß eine Verdrehbarkeit beispielsweise in einem Winkelbereich von +/- 30° möglich ist, vorzugsweise +/- 15°.

Eine optimale Anpassung zur Verbindung unterschiedlicher Knochenteile oder von Knochenfragmenten läßt sich erreichen, wenn die dritte Drehachse quer zur ersten und/oder zur zweiten Drehachse verläuft.

Damit die Fixationsvorrichtung am Verankerungselement erst nach Verankern des Verankerungselement in oder an einem Knochen festgelegt werden kann, ist es günstig, wenn der Verbindungsabschnitt das proximale Ende des Verankerungselements bildet oder im Bereich des proximalen Ende des Verankerungselements angeordnet ist. So ist es möglich, die Fixationsvorrichtung beispielsweise auf den Verbindungsabschnitt nach Einsetzen des Verankerungselements aufzusetzen.

Grundsätzlich wäre es denkbar, die Verbindungsabschnittaufnahme derart auszubilden, daß ein zylindrischer Verbindungsabschnitt aufgenommen werden kann. Um die Einstellbarkeit der Fixationsvorrichtung und damit des Verbindungselements relativ zum Verankerungselement noch weiter zu erhöhen, ist es vorteilhaft, wenn die Verbindungsabschnittaufnahme in Form einer Kugelgelenkaufnahme ausgebildet ist zum Aufnehmen eines kugeligen Verbindungsabschnitts.

Damit das Spannglied auf einfache Weise bewegt werden kann, ist es vorteilhaft, wenn es eine Werkzeugaufnahme aufweist zum Aufnehmen eines Spannwerkzeugs und wenn mit dem Spannwerkzeug das Spannglied bewegbar ist.

Die eingangs gestellte Aufgabe wird ferner bei einem orthopädischen Fixationssystem der eingangs beschriebenen Art erfindungsgemäß dadurch gelöst, daß die mindestens eine orthopädische Fixationsvorrichtung eine der oben beschriebenen Fixationsvorrichtungen ist. Wie bereits dargelegt, kann mit einer solchen Fixationsvorrichtung in einem einzigen Schritt die Fixationsvorrichtung von der Justierstellung in die Fixierstellung überführt werden. Abhängig von der besonderen Ausgestaltung der Fixationsvorrichtung weist dann das gesamte orthopädische Fixationssystem die oben erläuterten Vorteile auf.

Um die Fixationsvorrichtung auf einfache Weise mit dem Verankerungselement verbinden zu können, ist es vorteilhaft, wenn der Verbindungsabschnitt gewindefrei und zylindrisch oder im wesentlichen zylindrisch geformt ist. In der Justierstellung kann die Fixationsvorrichtung so auf einfache Weise relativ zum Verankerungselement verdreht werden, da der Verbindungsabschnitt so eine nahezu perfekte Lagerwelle bildet.

Um ein Kugelgelenk mit der Fixationsvorrichtung ausbilden zu können, ist es günstig, wenn der Verbindungsabschnitt des Verankerungselements kalottenförmig oder im wesentlichen kalottenförmig ist.

Gemäß einer bevorzugten Ausführungsform des Fixationssystems kann vorgesehen sein, daß das Verankerungselement einen sein distales Ende bildenden Schraubengewindeabschnitt zum Einschrauben in einen Knochen umfaßt. Ferner kann es vorteilhaft sein, wenn das Verankerungselement einen sein distales Ende bildenden Haken zum Verankern des Verankerungselements in einem Knochen umfaßt.

Vorzugsweise ist das Verbindungselement ein Stab oder eine Verbindungsplatte mit mindestens einem stabförmigen Plattenabschnitt. Derartige Verbindungselemente lassen sich auf einfache Weise mittels einer erfindungsgemäßen Fixationsvorrichtung mit einem Verankerungselement beliebiger Art verbinden.

Die nachfolgende Beschreibung bevorzugter Ausführungsformen der Erfindung dient im Zusammenhang mit der Zeichnung der näheren Erläuterung. Es zeigen:
- Figur 1:: eine perspektivische Gesamtansicht eines ersten Ausführungsbeispiels eines erfindungsgemäßen Fixationssystems;
- Figur 2:: eine teilweise zerlegte Fixationsvorrichtung in der Fixierstellung;
- Figur 3:: eine Explosionsdarstellung des in Figur 1 dargestellten Fixationssystems;
- Figur 4:: eine weitere perspektivische Ansicht der Explosionsdarstellung in Figur 3;
- Figur 5:: eine Schnittansicht längs Linie 5-5 in Figur 1;
- Figur 6:: eine Schnittansicht ähnlich Figur 5 durch ein zweites Ausführungsbeispiel einer Fixationsvorrichtung in der Justierstellung;
- Figur 7:: eine Ansicht ähnlich Figur 6 des zweiten Ausführungsbeispiels, jedoch in der Fixierstellung;
- Figur 8:: eine Schnittansicht ähnlich Figur 5 durch ein drittes Ausführungsbeispiel einer Fixationsvorrichtung;
- Figur 9:: eine Schnittansicht ähnlich Figur 5 durch ein viertes Ausführungsbeispiel einer Fixationsvorrichtung;
- Figur 10:: eine Schnittansicht ähnlich Figur 5 durch ein fünftes Ausführungsbeispiel einer Fixationsvorrichtung;

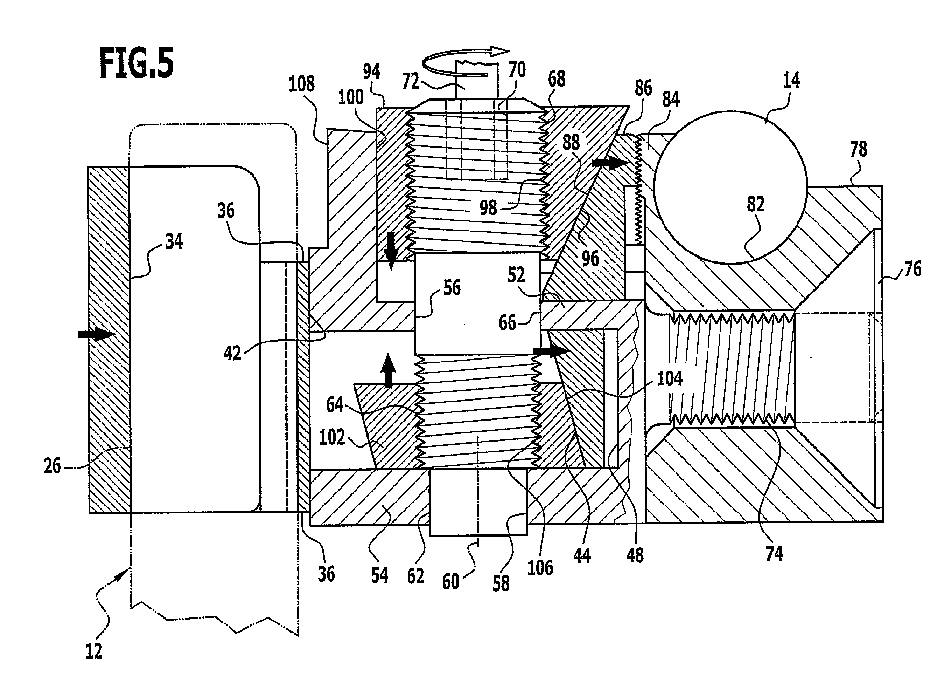

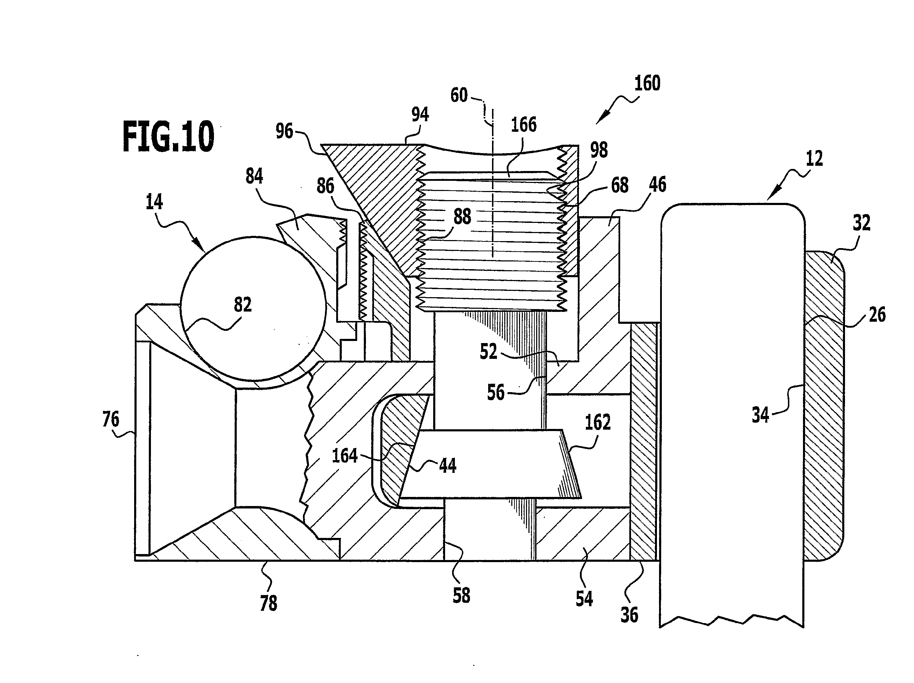

- Figur 10:: eine Schnittansicht ähnlich Figur 5 durch ein sechstes Ausführungsbeispiel einer Fixationsvorrichtung;

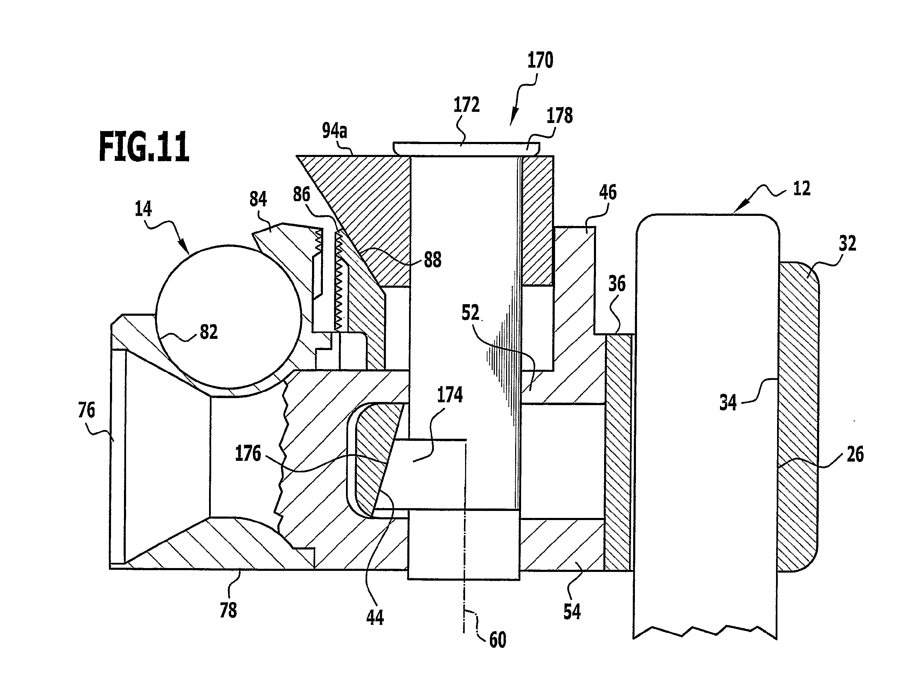

- Figur 11:: eine Schnittansicht ähnlich Figur 5 durch ein siebtes Ausführungsbeispiel einer Fixationsvorrichtung;

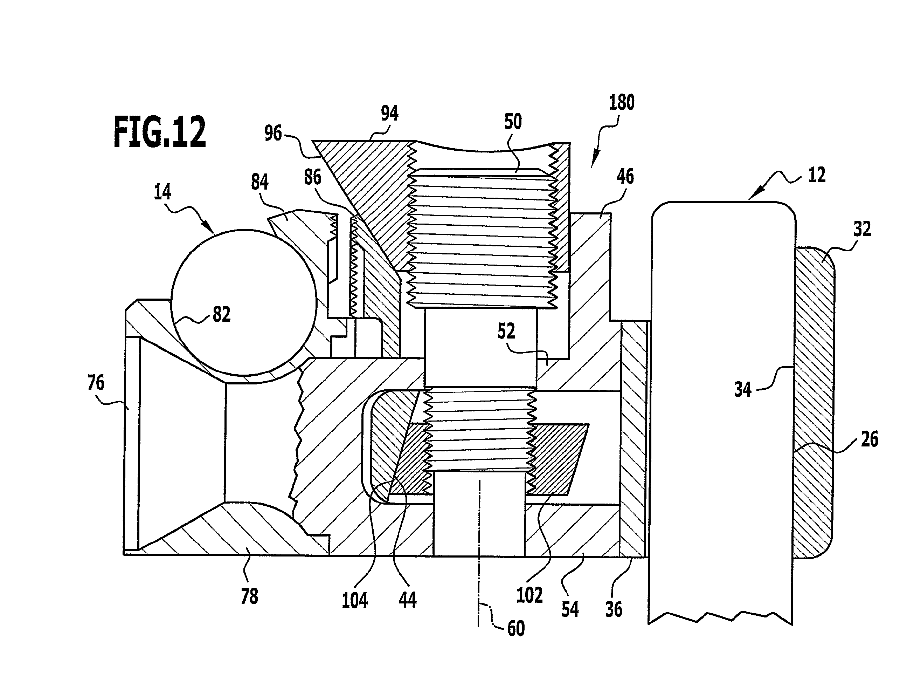

- Figur 12:: eine Schnittansicht ähnlich Figur 5 durch ein achtes Ausführungsbeispiel einer Fixationsvorrichtung; und

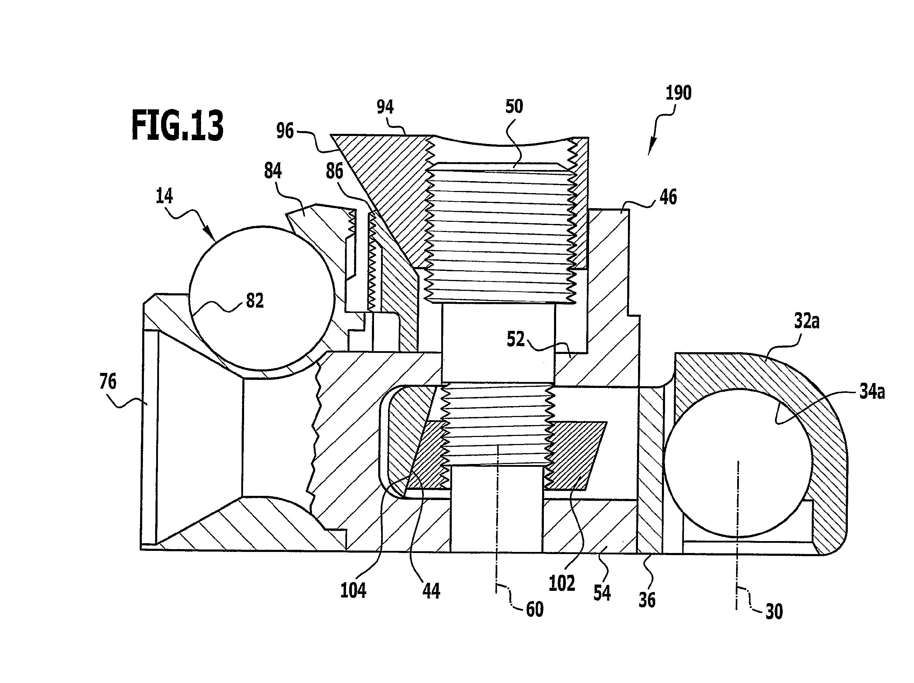

- Figur 13:: eine Schnittansicht ähnlich Figur 5 durch ein neuntes Ausführungsbeispiel einer Fixationsvorrichtung.

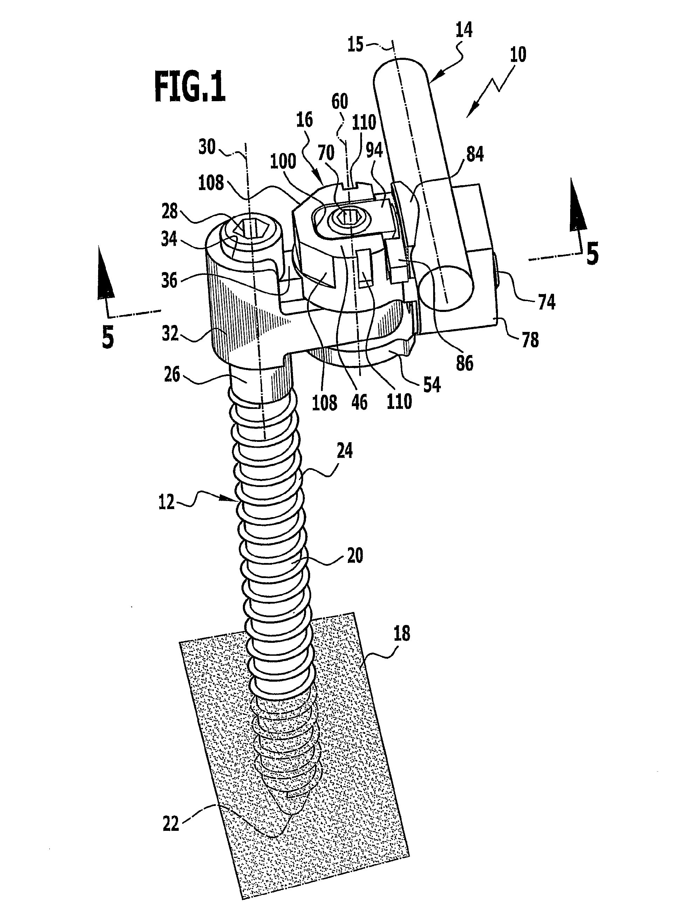

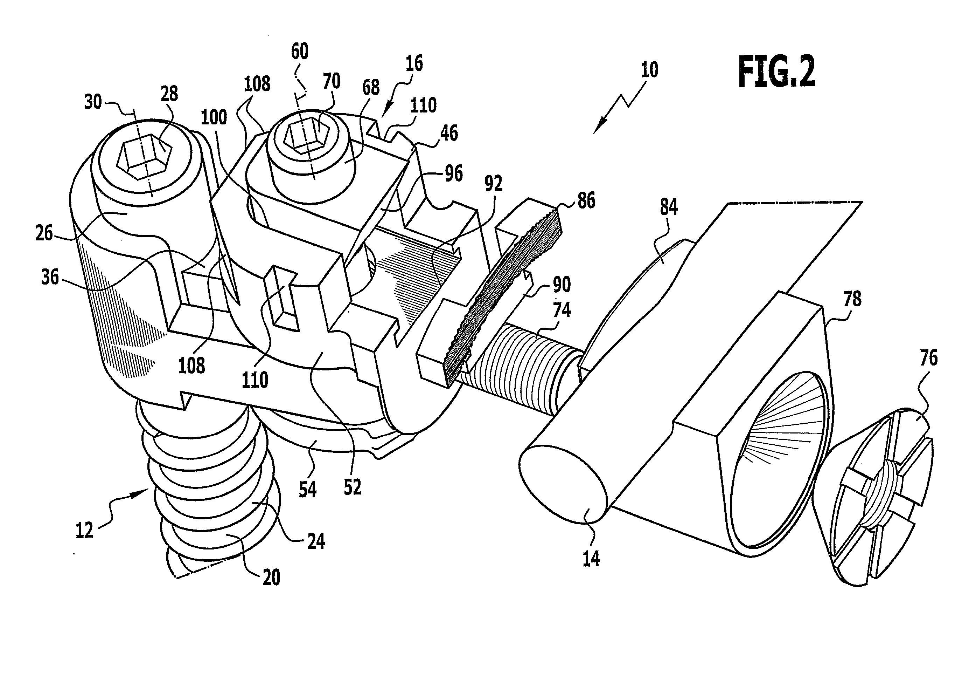

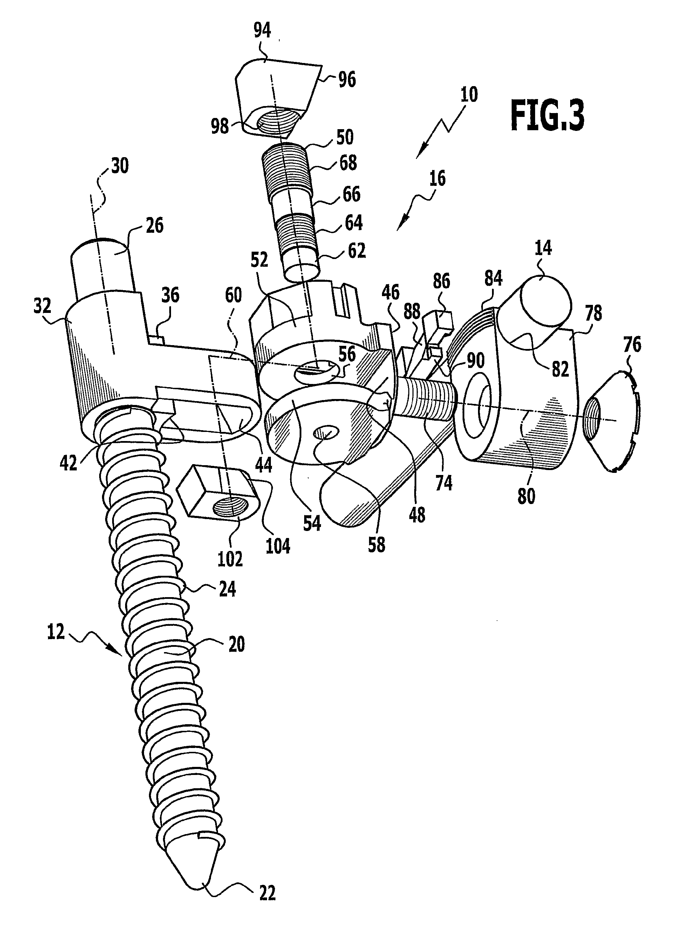

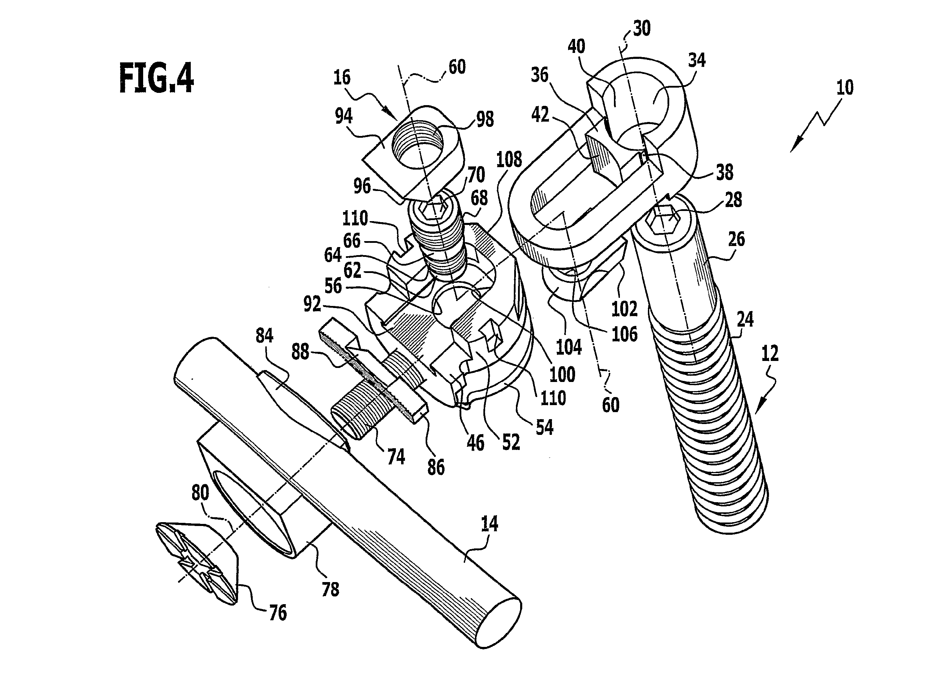

In den Figuren 1 bis 5 ist beispielhaft ein Teil eines insgesamt mit dem Bezugszeichen 10 versehenen Fixationssystems mit einem Verankerungselement in Form einer Knochenschraube 12, einem Verbindungselement in Form eines eine Längsachse 15 definierenden Stabes 14 sowie einem ersten Ausführungsbeispiel eines erfindungsgemäßen Klemmverbinders 16 dargestellt, mit dem der Stab 14 an der Knochenschraube 12 in einer Fixierstellung festgelegt werden kann.

Mit dem Fixationssystem lassen sich unterschiedliche Knochen oder Knochenfragmente 18 relativ zueinander positionieren und fixieren, indem in das jeweilige Knochenfragment 18 gleiche oder ähnliche Knochenschrauben 12 eingedreht und mittels des Stabs 14 miteinander verbunden werden, insbesondere durch Verwendung des Klemmverbinders 16 oder ähnlicher Verbindungselemente. Grundsätzlich könnte der Stab 14 an dem zweiten, nicht dargestellten Knochenfragment auch über eine völlig anders ausgestaltete Knochenschraube mit daran angeordnetem Klemmteil festgelegt werden.

Die Knochenschraube 12 weist einen langgestreckten zylindrischen Schaft 20 auf, welcher ausgehend von einer Spitze 22 auf etwa dreiviertel seiner Gesamtlänge mit einem Außengewinde 24 zum Einschrauben in das Knochenfragment 18 versehen ist. Ein proximales Ende der Knochenschraube 12 bildet einen zylindrischen Verbindungsabschnitt 26, welcher eine glatte äußere zylindrische Oberfläche aufweist. Zum Eindrehen der Knochenschraube 12 in das Knochenfragment 18 ist eine Werkzeugelementaufnahme in Form eines Innensechskant 28 vorgesehen, welcher symmetrisch zu einer Längsachse der Knochenschraube 12 ausgebildet ist.

Der Stab 14 hat eine geeignete Länge, um zwei Verankerungselemente miteinander zu verbinden und weist einen kreisrunden Querschnitt auf.

Der Klemmverbinder 16 umfaßt eine Offset-Brücke 32, welche eine im wesentlichen hohlzylindrische Verbindungsabschnittaufnahme 34 aufweist, welche auf den Verbindungsabschnitt 26 parallel zur Längsachse 30 in distaler Richtung der Knochenschraube aufgeschoben werden kann. Die Offset-Brücke 32 ist insgesamt im wesentlichen in Form einer ovalen Hülse ausgebildet, welche ihre größte Höhe im Bereich der Verbindungsabschnittaufnahme 34 aufweist. Im zusammengefügten Zustand wird der Verbindungsabschnitt 26 auf etwa 220° seines Umfangs von einem ersten gekrümmten Wandabschnitt der oralen Hülse umgeben, deren Höhe abgesehen von dem ersten gekrümmten Wandabschnitt im übrigen Bereich nur etwa die Hälfte der Gesamthöhe im Bereich der Verbindungsabschnittaufnahme 34 beträgt.

Auf die Längsachse 30 hin weisend liegt am Verbindungsabschnitt 26 in Richtung auf den in seiner Höhe niedrigeren Abschnitt der Offset-Brücke 32 ein Klemmstück 36 an, welches über einen schmalen, sich parallel zur Längsachse 30 erstreckenden Steg 38 mit dem die Verbindungsabschnittaufnahme 34 begrenzenden Teil der Offset-Brücke 32 federnd verbunden ist. Das Klemmstück 36 ist im wesentlichen quaderförmig ausgebildet und weist eine in Richtung auf den Verbindungsabschnitt 36 hin weisende konkave Anlagefläche 40 und eine in entgegengesetzter Richtung weisende Druckfläche 42 auf. Das Klemmstück 36 erstreckt sich parallel zur Längsachse auf einer Länge, die größer ist als eine Höhe des niedrigeren Abschnitts der Offset-Brücke 32, sodaß das Klemmstück 36 beidseitig etwas über eine Wand der Offset-Brücke 32 hervorsteht. Ferner ist eine Innenfläche der ovalen Offset-Brücke 32 in einem zweiten Krümmungsbereich, welcher auf die Verbindungsabschnittaufnahme 34 hin weist relativ zur Längsachse 30 um etwa 15° geneigt, und zwar in distaler Richtung von der Längsachse 30 weg weisend. Die geneigte Fläche bildet einen konischen Betätigungsflächenabschnitt 44.

Der von der Verbindungsabschnittaufnahme 34 weg weisende Bereich der Offset-Brücke 32 ist in eine U-förmige Aufnahme 48 eines Gelenkteils 46 eingesetzt und mittels eines Spannglieds 50 gesichert. Zu diesem Zweck sind eine obere Seitenwand 52 und eine untere Seitenwand 54, die die Aufnahme 48 seitlich begrenzen, mit konzentrischen Bohrungen 56 und 58 versehen, wobei die in der unteren Seitenwand 54 vorgesehene Bohrung 58 im Durchmesser kleiner ist als die Bohrung 56 in der oberen Seitenwand 52. Das Spannglied 50 definiert mit seiner Längsachse 60 eine erste Drehachse, um die das Gelenkteil 46 relativ zur Offset-Brücke 32 verschwenkt werden kann.

Das Spannglied 50 ist in insgesamt vier Abschnitt unterteilt, nämlich einen ersten, zur Bohrung 58 korrespondierenden zylindrischen Lagerabschnitt 62 und einen sich daran anschließenden Rechtsgewindeabschnitt 64, welcher im Außendruchmesser etwas größer ist als der Lagerabschnitt 62. An den Rechtsgewindeabschnnitt 64 schließt sich ein zweiter zylindrischer, zur Bohrung 56 korrespondierender Abschnitt 66 an, dessen Außendurchmesser etwas größer ist als der des Rechtsgewindeabschnitts 64. Ein dem Lagerabschnitt 62 gegenüberliegendes Ende des Spannglieds 50 wird durch einen Linksgewindeabschnitt 68 gebildet, welcher einen gegenüber dem zylindrischen Abschnitt 66 etwas vergrößerten Außendurchmesser aufweist. Durch diese Ausgestaltung kann das Spannglied 50 mit dem Lagerabschnitt 62 voran durch die Bohrung 56 hindurchgesteckt werden, wobei nach dem Zusammenbau des Klemmverbinders 16 der Lagerabschnitt 62 und der zylindrische Abschnitt 66 jeweils in den Bohrungen 56 beziehungsweise 58 geführt werden. Konzentrisch zur Längsachse 60 ist im Bereich des Linksgewindeabschnitts 68 eine Werkzeugelementaufnahme in Form eines Innensechskants 70 vorgesehen zur Aufnahme eines Spannwerkzeugs 72, dessen distales Ende in Figur 5 schematisch dargestellt ist. Mit dem Spannwerkzeug 72 kann das Spannglied 50 um seine Längsachse 60 verdreht werden.

Quer zur Längsachse und entgegengesetzt zur der Aufnahme 48 weisend steht am Gelenkteil 46 ein Gewindebolzen 74 ab, auf welchen mittels einer Mutter 76 eine Klemmbacke 78 gesichert ist. Eine Längsachse 80 des Gewindebolzens 74 verläuft quer zur Längsachse 60. An der Klemmbacke 78 ist eine Verbindungselementaufnahme in Form einer im wesentlichen hohlzylindrischen Stabaufnahme 82 vorgesehen, in welche der Stab 14 eingeschoben oder auch quer zu seiner Längsachse eingeklickt werden kann. Zu diesem Zweck ist die Stabaufnahme 82 über einen ausreichenden Winkelbereich parallel zur ihrer Längsachse 15 geöffnet. Die Stabaufnahme 82 weist einen in Richtung auf die Längsachse 60 hin weisenden Klemmabschnitt 84 auf, welcher durch Materialschwächung etwas federn kann. Am Klemmabschnitt 84 liegt ein Klemmteil 86 an, welches eine um etwa 25° relativ zur Längsachse 60 geneigte ebene Betätigungsfläche 86 aufweist. Das Klemmteil 86 ist am Gelenkteil 46 parallel zur Längsachse 80 verschiebbar gelagert, und zwar indem ein T-förmiger Vorsprung 90 des Klemmteils 86 in einer T-Nut 92 in der oberen Seitenwand 52 geführt ist.

Zum klemmenden Spannen des Stabes 14 in der Stabaufnahme 82 ist ein erstes Klemmteil 94 vorgesehen, welches im wesentlichen quaderförmig ausgebildet ist, wobei eine Stirnfläche korrespondierend zur Betätigungsfläche 88 abgeschrägt ist und eine Aufgleitfläche 96 bildet. Das erste Klemmteil 94 ist mit einer ein zum Linksgewindeabschnitt 68 korrespondierendes Innengewinde 98 aufweisenden Bohrung versehen und in einer U-förmigen Ausnehmung 100 auf einer Oberseite der oberen Seitenwand 52 geführt und dadurch gegen ein Verdrehen um die Längsachse 60 gesichert.

Ferner ist ein zweites Klemmteil 102 vorgesehen, welches in Form eines Parallelepipeds ausgebildet ist und eine zum konischen Betätigungsflächenabschnitt 44 korrespondierende zweite Aufgleitfläche 104 aufweist. Ferner ist das zweite Klemmteil 102 mit einer mit einem Innengewinde 106 versehenen Bohrung versehen, wobei das Innengewinde 106 korrespondierend zum Rechtsgewindeabschnitt 64 ausgebildet ist.

Des weiteren sind an einer Außenwand der U-förmigen Ausnehmung 100 jeweils unter einem Winkel von 45° gegeneinander geneigte Abflachungen 108 vorgesehen sowie zwei diametral gegenüberliegende Vertiefungen 110. Die Abflachungen 108 und die Vertiefungen 110 dienen der Aufnahme eines nicht dargestellten Einsetzwerkzeugs für den Klemmverbinder 16.

Die Funktionsweise des Fixationssystems 10, insbesondere des Klemmverbinders 16 wird mit Bezug zur Figur 5 nachfolgend näher erläutert.

Der Klemmverbinder 16 ist in Figur 5 in der sogenannten Justierstellung dargestellt. Dies bedeutet, daß der Verbindungsabschnitt 26 in die Verbindungsabschnittaufnahme 34 eingeführt werden kann, was gleichzeitig eine Rotation der Offset-Brücke 32 um die Längsachse 30 gestattet. Ferner kann das Gelenkteil 46 relativ zur Offset-Brücke 32 um die Längsachse 60 verschwenkt werden. Des weiteren kann der Stab 14 in der Stabaufnahme 82 verschoben werden.

Nachdem die Knochenschraube 12 in gewünschter Weise im Knochenfragment 18 verankert ist, wird der Klemmverbinder 16 auf die Knochenschraube 12 aufgesetzt und der Stab 14 in die Stabaufnahme 82 eingelegt. Ein Operateur kann nun eine Relativposition des Stabes 14 zur Knochenschraube 12 in gewünschter Weise einstellen. Dabei hat er auch die Möglichkeit, eine Neigung der Längsachse 15 des Stabes 14 relativ zu den Längsachsen 30 und 60 zu variieren, und zwar durch Verschwenken der Klemmbacke 78 um die Längsachse 80. Hier sind nicht näher dargestellte seitliche Anschläge vorgesehen, die einen Schwenkbereich der Klemmbacke 78 um die Längsachse 80 auf etwa +/- 15° begrenzen.

Zum Festlegen des Stabs 14 an der Knochenschraube 12 wird mittels des Spannwerkzeugs 72 das Spannglied 52 im Uhrzeigersinn gedreht. Die Folge hiervon ist, daß das erste Klemmteil 94 in Richtung auf die obere Seitenwand 52 bewegt wird und daß das zweite Klemmteil 102 ebenfalls in Richtung auf die obere Seitenwand 52 bewegt wird. Insgesamt werden die beide Klemmteile 94 und 102 bei Rotation des Spannglieds im Uhrzeigersinn also aufeinander zu bewegt. Infolge der Bewegung des ersten Klemmteils 94 parallel zur Längsachse 60 gleitet die erste Aufgleitfläche 96 an der Betätigungsfläche 88 auf, wodurch das Klemmteil 86 in Richtung auf den Stab 14 bewegt wird, was durch einen Pfeil angedeutet ist, und den Klemmabschnitt 84 in Richtung auf den Stab 14 drückt. Dadurch wird der Stab 14 klemmend in der Stabaufnahme 82 festgelegt. In analoger Weise gleitet die zweite Aufgleitfläche 104 am konischen Betätigungsflächenabschnitt 44 auf, mit der Folge, daß die gesamte Offset-Brücke 32 in Richtung des Pfeils links in Figur 5 parallel zur Längsachse 80 gezogen wird. Die Druckfläche 42 liegt an abgerundeten Stirnflächen des Gelenkteils 46 an, sodaß das Klemmstück 36 relativ zum Verbindungsabschnitt 26 auf diesen hin bewegt wird, wodurch sich insgesamt ein Querschnitt der Verbindungsabschnittaufnahme 34 verringert und der Verbindungsabschnitt 26 klemmend gehalten wird.

Mit dem erfindungsgemäßen Klemmverbinder 16 ist es möglich, den Stab 14 um zwei parallel zueinander verlaufende Drehachsen, nämlich die Längsachsen 30 und 60, relativ zum Verbindungsabschnitt 26 zu verschwenken. Auf diese Weise ist eine Verschiebung des Stabes 14 parallel zu sich selbst in Richtung auf den Verbindungsabschnitt 26 hin möglich. Ferner kann allein durch Bewegen des Spannglieds 50 bewirkt werden, daß der Klemmverbinder 16 von einer Justierstellung, in welcher sowohl der Stab 14 als auch der Verbindungsabschnitt 26 lose am Klemmverbinder 16 gehalten sind, in die Fixierstellung überführt werden kann, in welcher sowohl der Stab 14 als auch der Verbindungsabschnitt 26 am Klemmverbinder 16 unbeweglich festgelegt sind. Zum Fixieren des Stabs 14 an der Knochenschraube 12 ist daher nur ein Verriegelungsschritt notwendig, der durch Drehen des Spannglieds 50 ausgeführt wird.

In umgekehrter Weise läßt sich der Klemmverbinder 16 auch wieder von der Fixierstellung in die Justierstellung überführen.

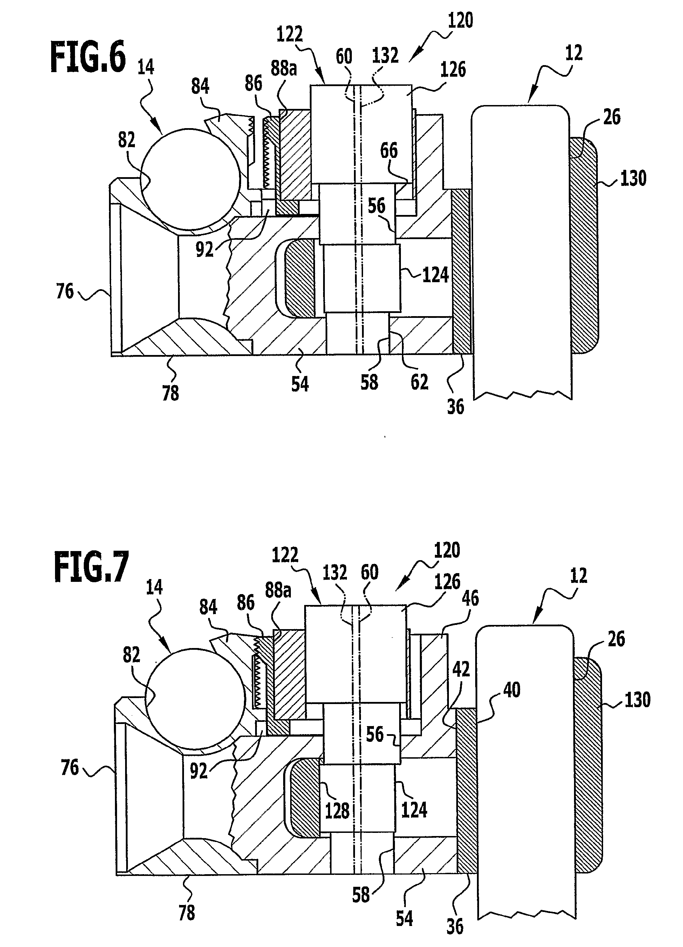

Nachfolgend werden im Zusammenhang mit den Figuren 6 bis 13 sieben weitere oder auch alternative Ausführungsformen des Klemmverbinders 16 näher beschrieben. Der Einfachheit halber sind im Vergleich zum Klemmverbinder 16 identische oder sehr ähnliche Teile mit denselben Bezugszeichen versehen. Abgewandelte Teile oder Elemente der Klemmverbinder sind explizit beschrieben und mit eigenständigen Bezugszeichen versehen.

Ein zweites Ausführungsbeispiel eines insgesamt mit dem Bezugszeichen 120 versehenen Klemmverbinders ist in den Figuren 6 und 7 dargestellt. Ein insgesamt mit dem Bezugszeichen 122 versehenes Spannglied unterscheidet sich vom Spannglied 50 dadurch, daß anstelle der beiden Gewindeabschnitte 64 und 68 benachbart dem Lagerabschnitt 62 beziehungsweise dem zylindrischen Abschnitt 66 jeweils ein exzentrisch zur Längsachse 60 angeordneter Zylinderabschnitt 124 beziehungsweise 126 vorgesehen ist. Des weiteren ist, anders als beim Klemmverbinder 16, anstelle des konischen Betätigungsflächenabschnitts 44 ein hohlzylindrischer Abschnitt 128 vorgesehen, an welchem eine Außenfläche des Zylinderabschnitts 124 infolge einer Drehung des Spannglieds 122 aufgleiten und die insgesamt mit dem Bezugszeichen 130 versehene Offset-Brücke 130 in Richtung auf den Stab 14 hin bewegen kann. In analoger Weise kann mittels des Zylinderabschnitts 126 das Klemmteil 86 mit seiner Betätigungsfläche 88a in Richtung auf den Klemmabschnitt 84 hin verschoben werden, wodurch der Stab 14 in der Stabaufnahme 82 klemmend gehalten werden kann. Es sind somit auch beim Klemmverbinder 120 zusammenwirkende Betätigungs- und Aufgleitflächen vorgesehen, wobei diese relativ zur Längsachse 60 nicht geneigt sind. Ein Aufgleiten wird aufgrund der Exzentrizität der Zylinderabschnitte 124 und 126 bewirkt, wobei die Exzentrizität zu erkennen ist anhand einer in den Figuren 6 und 7 parallel zur Längsachse 60 eingezeichneten Exzenterachse 132, welche eine Symmetrieachse der Zylinderabschnitten 124 und 126 bildet.

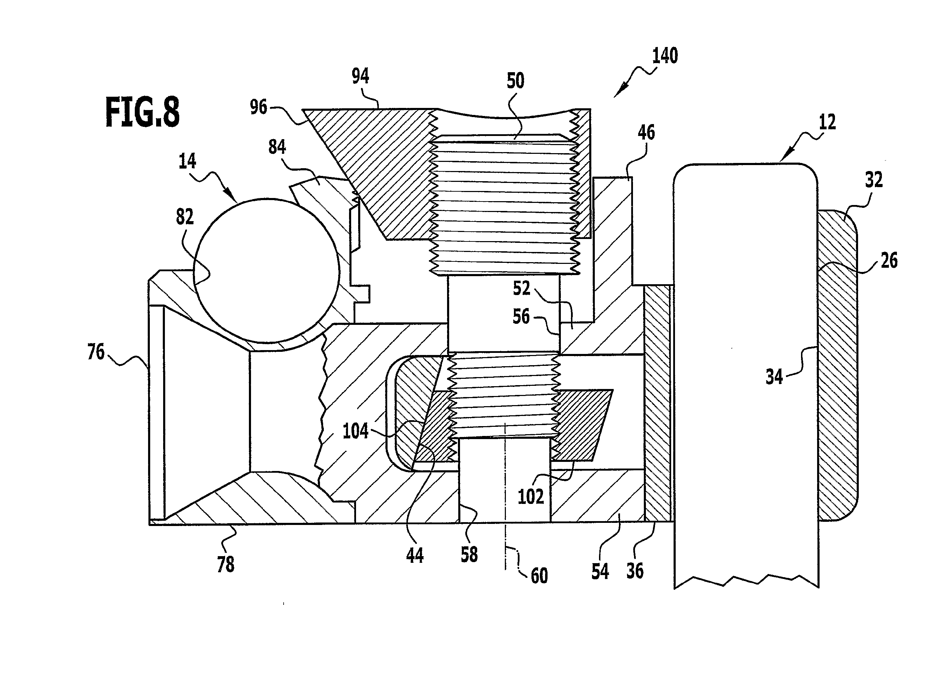

In Figur 8 ist ein drittes Ausführungsbeispiel eines insgesamt mit dem Bezugszeichen 140 versehenen Klemmverbinders dargestellt. Der Klemmverbinder 140 stimmt nahezu identisch mit dem Klemmverbinder 16 überein, allerdings wurde bei diesem auf das Klemmteil 86 verzichtet, sodaß die erste Aufgleitfläche 96 direkt am Klemmabschnitt 84 der Stabaufnahme 82 anliegt. Das erste Klemmteil 94 erstreckt sich daher etwas weiter in Richtung auf die Stabaufnahme 82 hin als beim Klemmverbinder 16. Ansonsten entspricht die Funktionsweise des Klemmverbinders 140 der im Zusammenhang mit dem Klemmverbinder 16 beschriebenen Funktionsweise.

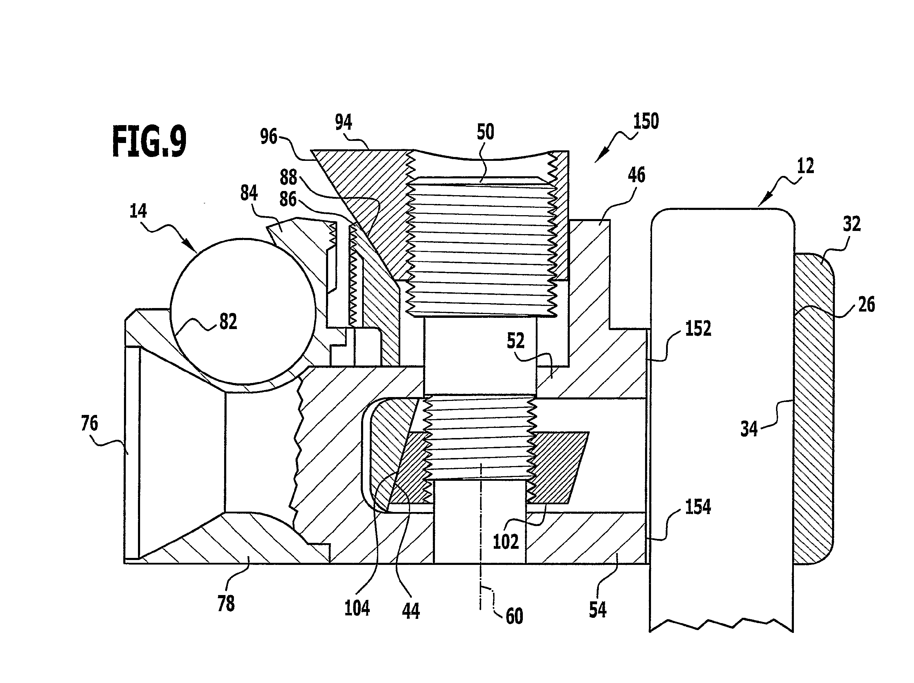

Ein viertes Ausführungsbeispiel eines Klemmverbinders ist in Figur 9 insgesamt mit dem Bezugszeichen 150 versehen. Er unterscheidet sich vom Klemmverbinder 16 lediglich dadurch, daß auf das Klemmstück 36 verzichtet wurde. Stirnflächen 152 und 154 der oberen Seitenwand 52 beziehungsweise der unteren Seitenwand 54 sind zu diesem Zweck konkav geformt und liegen direkt am Verbindungsabschnitt 26 der Knochenschraube 12 an. Der Verbindungsabschnitt 26 wird in der Fixierstellung zwischen den Stirnflächen 152 beziehungsweise 154 einerseits und einer diametral gegenüberliegenden Innenfläche der Verbindungsabschnittaufnahme 34 andererseits klemmend gehalten. Im übrigen entspricht die Funktionsweise des Klemmverbinders 150 derjenigen, die im Zusammenhang mit dem Klemmverbinder 16 näher erläutert wurde.

In Figur 10 ist ein insgesamt mit dem Bezugszeichen 160 versehener Klemmverbinder dargestellt, der sich vom Klemmverbinder 16 dadurch unterscheidet, daß anstelle des zweiten Klemmteils 102 ein Konus 162 vorgesehen ist, dessen geneigte konische Außenfläche 164 eine der zweiten Aufgleitfläche 104 entsprechende Funktion ausübt. Der Konus 162 ist an dem mit dem Bezugszeichen 166 versehenen Spannglied axial unbeweglich gesichert. Infolge einer Rotation des Spannglieds 166 im Uhrzeigersinn um die Längsachse 60 wird das erste Klemmteil 94 in Richtung auf den Konus 162 hin gezogen, wodurch die Offset-Brücke 32 in Richtung auf den Stab 14 hingezogen und dadurch klemmend in der Verbindungsabschnittaufnahme 34 eingespannt wird, der Stab 14 durch Druckausübung auf den Klemmabschnitt 84 in der Stabaufnahme 82.

Ein sechstes Ausführungsbeispiel eines insgesamt mit dem Bezugszeichen 170 versehenen Klemmverbinders ist in Figur 11 dargestellt. Anders als beim Klemmverbinder 16 dient hier ein nietartiges Spannglied 172 zum Überführen des Klemmverbinders 170 von der Justierstellung in die Fixierstellung. Anstelle des ersten Klemmteils 94 ist ein Klemmteil 94a vorgesehen, welches in seiner Form dem ersten Klemmteil 94 entspricht, jedoch kein Innengewinde aufweist. Am distalen Ende des Spannglieds 172, dem konischen Betätigungsflächenabschnitt 44 gegenüberliegend, ist ein zu diesem korrespondierender Vorsprung 174 mit einer zweiten Aufgleitfläche 176 vorgesehen, die am konischen Betätigungsflächenabschnitt 44 anliegt. Der Vorsprung 174 kann einstückig mit dem Spannglied 172 ausgebildet sein oder mit diesem fest verbunden. Zum Überführen des Klemmverbinders 170 von der Justierstellung in die Fixierstellung wird mit einem nicht näher dargestellten Spannwerkzeug das erste Klemmteil 94a parallel zur Längsachse 60 in Richtung auf die obere Seitenwand 52 gedrückt und gleichzeitig das Spannglied 172 etwas in entgegengesetzter Richtung gezogen. Sobald in ähnlicher Weise wie beim Klemmverbinder 16 der Verbindungsabschnitt 26 in der Verbindungsabschnittaufnahme 34 festgelegt ist und der Stab 14 in der Stabaufnahme 82, wird ein Kopf 178 des Spannglieds 172 derart verformt, daß das erste Klemmteil 94a in seiner an der Betätigungsfläche 88 aufgeglittenen Stellung festgelegt wird. Ein Lösen des Klemmverbinders 170 ist nur durch Zerstörung des Spannglieds 172 möglich. Mit dem Klemmverbinder 170 läßt sich daher einmalig eine Verbindung zwischen der Knochenschraube 12 und dem Stab 14 herstellen, die jedoch nicht mehr gelöst oder nachjustiert werden kann.

In Figur 12 ist ein insgesamt mit dem Bezugszeichen 180 versehenes achtes Ausführungsbeispiel eines Klemmverbinders dargestellt. Der Klemmverbinder 180 unterscheidet sich vom Klemmverbinder 16 dadurch, daß das Klemmstück 36 nicht einstückig mit der Offset-Brücke 32 verbunden ist, das heißt nicht über einen Steg mit dieser verbunden ist. Ansonsten stimmt der Aufbau mit dem des Klemmverbinders 16 und damit auch die prinzipielle Funktionsweise mit diesem überein.

Schließlich ist in Figur 13 ein insgesamt mit dem Bezugszeichen 190 versehenes achtes Ausführungsbeispiel eines erfindungsgemäßen Klemmverbinders dargestellt. Es unterscheidet sich vom Klemmverbinder 16 lediglich durch die Ausgestaltung der Verbindungsabschnittaufnahme 34, die beim Klemmverbinder 190 mit dem Bezugszeichen 34a versehen ist. Sie bildet einen kalottenförmigen Hohlraum zur Aufnahme eines kalottenförmigen Endes einer nicht näher dargestellten Knochenschraube. Der Klemmverbinder 190 kann also dazu verwendet werden, einen Stab 14 an einer Knochenschraube mit einem Kugelkopf festzulegen. In analoger Weise wie bei allen anderen Klemmverbindern kann allein durch Bewegen des Spannglieds 50 der Klemmverbinder 190 sowohl relativ zum Stab 14 als auch zur nicht dargestellten Knochenschraube festgelegt werden.

Zum Klemmverbinder 190 sei angemerkt, daß auch die oben beschriebenen Klemmverbinder 120, 140, 150, 160, 170 und 180 mit einer kalottenförmigen Verbindungsabschnittaufnahme 34a versehen sein können.

Ferner besteht auch die Möglichkeit, bei den Klemmverbindern 120, 140, 150, 160, 170 und 190 auf das Klemmstück 36, wie im Zusammenhang mit dem Klemmverbinder 180 beschrieben, zu verzichten. Des weiteren kann auch bei den Klemmverbindern 120, 150, 160, 170, 180 und 190 auf das Klemmteil 86 verzichtet werden.

## Patentansprüche

1. Orthopädische Fixationsvorrichtung (16; 120; 140; 150; 160; 170; 180; 190) zum Verbinden eines in oder an einem Knochen (18) verankerbaren, einen Verbindungsabschnitt (26) aufweisenden ersten Verankerungselements (12) mit einem Verbindungselement (14), welches mit einem zweiten, in oder an einem Knochen verankerbaren Verankerungselement verbindbar ist, wobei die Fixationsvorrichtung (16; 120; 140; 150; 160; 170; 180; 190) in einer Justierstellung relativ zum Verbindungselement (14) und zum Verankerungselement (12) bewegbar ist und in einer Fixierstellung am Verbindungselement (14) und am Verankerungselement (12) festlegbar ist, wobei ein einziges beweglich gelagertes Spannglied (50; 122; 166; 172) vorgesehen ist zum Überführen der Fixationsvorrichtung (16; 120; 140; 150; 160; 170; 180; 190) von der Justierstellung und in die Fixierstellung und umgekehrt, **dadurch gekennzeichnet, daß** eine erste Klemmvorrichtung vorgesehen ist zum klemmenden Festlegen der Fixationsvorrichtung (16; 120; 140; 150; 160; 170; 180; 190) am Verbindungselement (14) in der Fixierstellung, daß eine zweite Klemmvorrichtung vorgesehen ist zum klemmenden Festlegen der Fixationsvorrichtung (16; 120; 140; 150; 160; 170; 180; 190) am Verankerungselement (12) in der Fixierstellung und daß die erste Klemmvorrichtung das Spannglied (50; 122; 166; 172) umfaßt und daß die zweite Klemmvorrichtung das Spannglied (50; 122; 166; 172) umfaßt.

2. Fixationsvorrichtung nach Anspruch 1, **dadurch gekennzeichnet, daß** die Fixationsvorrichtung (16; 120; 140; 150; 160; 170; 180; 190) mindestens ein erstes Trägerelement (46) umfaßt und daß das Spannglied (50; 122; 166; 172) rotierbar an dem mindestens einen ersten Trägerelement (46) gelagert ist.

3. Fixationsvorrichtung nach einem der voranstehenden Ansprüche, **dadurch gekennzeichnet, daß** die Fixationsvorrichtung (16; 120; 140; 150; 160; 170; 180; 190) mindestens ein erstes Trägerelement (46) umfaßt und daß das Spannglied (50; 122; 166; 172) relativ zum ersten Trägerelement (46) translatierbar gelagert ist.

4. Fixationsvorrichtung nach einem der voranstehenden Ansprüche, **dadurch gekennzeichnet, daß** das Verbindungselement (14) in der Justierstellung relativ zum Verankerungselement (12) parallel zu sich selbst auf das Verankerungselement (12) hin oder von diesem weg verschiebbar ist.

5. Fixationsvorrichtung nach einem der voranstehenden Ansprüche, **dadurch gekennzeichnet, daß** die Fixationsvorrichtung (16; 120; 140; 150; 160; 170; 180; 190) eine zum Verbindungselement (14) korrespondierende Verbindungselementaufnahme (82) zum Aufnehmen des Verbindungselements (14) aufweist.

6. Fixationsvorrichtung nach einem der voranstehenden Ansprüche, **dadurch gekennzeichnet, daß** die Fixationsvorrichtung (16; 120; 140; 150; 160; 170; 180; 190) eine zum Verbindungsabschnitt (26) korrespondierende Verbindungsabschnittaufnahme (34) zum Aufnehmen des Verbindungsabschnitts (26) aufweist.

7. Fixationsvorrichtung nach Anspruch 5 und 6, **dadurch gekennzeichnet, daß** die Verbindungsabschnittaufnahme (34) relativ zur Verbindungselementaufnahme (82) um eine erste Drehachse (60) verdrehbar ist.

8. Fixationsvorrichtung nach Anspruch 5 und 6 oder nach Anspruch 7,
**dadurch gekennzeichnet, daß** die Fixationsvorrichtung (16; 120; 140; 150; 160; 170; 180; 190) ein die Verbindungselementaufnahme (82) tragendes erstes Trägerelement (46) und ein am ersten Trägerelement (46) in der Justierstellung um eine erste Drehachse (60) drehbar gelagertes zweites, die Verbindungsabschnittaufnahme (34) tragendes Trägerelement (32) umfaßt.

9. Fixationsvorrichtung nach Anspruch 7 oder 8, **dadurch gekennzeichnet, daß** das Spannglied (50; 122; 166; 172) eine die erste Drehachse (60) definierende Lagerwelle bildet.

10. Fixationsvorrichtung nach einem der Ansprüche 8 oder 9, **dadurch gekennzeichnet, daß** die erste Klemmvorrichtung ein erstes Klemmelement (94; 126) umfaßt, welches eine an einer ersten Betätigungsfläche eines ersten Klemmstücks (84) des ersten Trägerelements (46) direkt oder indirekt anliegende erste Aufgleitfläche (96) aufweist, und daß infolge einer Bewegung des Spannglieds (50; 122; 166; 172) das erste Klemmelement (94; 126) derart bewegbar ist, daß die erste Aufgleitfläche (96) an der ersten Betätigungsfläche aufgleiten und einen Querschnitt der Verbindungselementaufnahme (82) derart verringern kann, sodaß das Verbindungselement (14) in der Verbindungselementaufnahme (82) festgelegbar ist.

11. Fixationsvorrichtung nach Anspruch 10, **dadurch gekennzeichnet, daß** ein erstes, zwischen der ersten Aufgleitfläche (96) und der ersten Betätigungsfläche angeordnetes Kraftübertragungsglied (86) vorgesehen ist zum Übertragen einer Kraft vom ersten Klemmelement (94; 126) auf das erste Klemmstück (84).

12. Fixationsvorrichtung nach Anspruch 10 oder 11, **dadurch gekennzeichnet, daß** die erste Aufgleitfläche (96) und die erste Betätigungsfläche (88) relativ zur ersten Drehachse (60) geneigt sind.

13. Fixationsvorrichtung nach Anspruch 10 oder 11, **dadurch gekennzeichnet, daß** die erste Aufgleitfläche eine erste Außenfläche eines um die erste Drehachse (60) verdrehbaren ersten Exzenters (126) ist und daß die erste Betätigungsfläche (88a) mindestens abschnittsweise konkav in Richtung auf die erste Drehachse (60) hin gekrümmt ist.

14. Fixationsvorrichtung nach einem der Ansprüche 10 bis 13, **dadurch gekennzeichnet, daß** das erste Klemmelement (94) in Form eines Konus ausgebildet ist.

15. Fixationsvorrichtung nach einem der Ansprüche 10 bis 14, **dadurch gekennzeichnet, daß** das erste Klemmelement und das Spannglied (50; 122; 166; 172) einstückig ausgebildet sind.

16. Fixationsvorrichtung nach einem der Ansprüche 10 bis 14, **dadurch gekennzeichnet, daß** das erste Klemmelement (94; 126) und das Spannglied (50; 122; 166; 172) zweiteilig ausgebildet sind.

17. Fixationsvorrichtung nach Anspruch 16, **dadurch gekennzeichnet, daß** das erste Klemmelement und das Spannglied (50; 122; 166; 172) drehfest miteinander verbunden sind.

18. Fixationsvorrichtung nach einem der Ansprüche 10 bis 17, **dadurch gekennzeichnet, daß** das Spannglied (50; 166) einen ersten Außengewindeabschnitt (68) aufweist, daß das erste Klemmelement (94) einen zum ersten Außengewindeabschnitt (68) korrespondierenden ersten Innengewindeabschnitt (98) aufweist und daß durch Drehen des Spannglieds (50; 166) um seine Längsachse das erste Klemmelement (94) parallel zu sich selbst verschiebbar ist.

19. Fixationsvorrichtung nach einem der Ansprüche 8 bis 18, **dadurch gekennzeichnet, daß** die zweite Klemmvorrichtung ein zweites Klemmelement (102; 124; 162; 174) umfaßt, welches eine an einer zweiten Betätigungsfläche (44; 128) des zweiten Trägerelements (32) direkt oder indirekt anliegende zweite Aufgleitfläche (104; 164) aufweist, und daß infolge einer Bewegung des Spannglieds (50; 122; 166; 172) das zweite Klemmelement (102; 124; 162; 174) derart bewegbar ist, daß die zweite Aufgleitfläche (104; 164) an der zweiten Betätigungsfläche (44; 128) aufgleiten und ein die Verbindungsabschnittaufnahme abschnittsweise begrenzendes zweites Klemmstück (36; 152, 154) derart bewegbar ist, daß sich ein Querschnitt der Verbindungsabschnittaufnahme (34) verringern kann, sodaß der Verbindungsabschnitt (26) des Verankerungselements (12) in der Verbindungsabschnittaufnahme (34) festlegbar ist.

20. Fixationsvorrichtung nach Anspruch 19, **dadurch gekennzeichnet, daß** die zweite Aufgleitfläche (104; 164) und die zweite Betätigungsfläche (44) relativ zur ersten Drehachse (60) geneigt sind.

21. Fixationsvorrichtung nach einem der Ansprüche 19 oder 20, **dadurch gekennzeichnet, daß** die zweite Aufgleitfläche eine zweite Außenfläche eines um die erste Drehachse (60) verdrehbaren zweiten Exzenters (124) ist und daß die zweite Betätigungsfläche (128) mindestens abschnittsweise konkav in Richtung auf die erste Drehachse (60) hin gekrümmt ist.

22. Fixationsvorrichtung nach einem der Ansprüche 19 oder 20, **dadurch gekennzeichnet, daß** das zweite Klemmelement (102; 162; 174) mindestens teilweise in Form eines Konus ausgebildet ist.

23. Fixationsvorrichtung nach einem der Ansprüche 19 bis 22, **dadurch gekennzeichnet, daß** das zweite Klemmelement (166; 172) und das Spannglied (162; 174) einstückig ausgebildet sind.

24. Fixationsvorrichtung nach einem der Ansprüche 19 bis 22 **dadurch gekennzeichnet, daß** das zweite Klemmelement (102; 162) und das Spannglied (50; 166) zweiteilig ausgebildet sind.

25. Fixationsvorrichtung nach Anspruch 24, **dadurch gekennzeichnet, daß** das zweite Klemmelement (162; 174) und das Spannglied (50; 166) drehfest miteinander verbunden sind.

26. Fixationsvorrichtung nach einem der Ansprüche 17 bis 25, **dadurch gekennzeichnet, daß** das Spannglied (50) einen zweiten Außengewindeabschnitt (64) aufweist, daß das zweite Klemmelement (102) einen zum zweiten Außengewindeabschnitt (64) korrespondierenden zweiten Innengewindeabschnitt (106) aufweist und daß durch Drehen des Spannglieds (50) um seine Längsachse (60) das zweite Klemmelement (102) parallel zu sich selbst verschiebbar ist.

27. Fixationsvorrichtung nach Anspruch 26, **dadurch gekennzeichnet, daß** der erste und der zweite Außengewindeabschnitt (64, 68) gegenläufige Gewindeabschnitte sind.

28. Fixationsvorrichtung nach Anspruch 27, **dadurch gekennzeichnet, daß** der erste Außengewindeabschnitt ein Rechtsgewindeabschnitt (64) und daß der zweite Außengewindeabschnitt ein Linksgewindeabschnitt (68) ist.

29. Fixationsvorrichtung nach einem der Ansprüche 19 bis 28, **dadurch gekennzeichnet, daß** sich in der Fixierstellung das zweite Trägerelement (32) über das zweite Klemmstück (36) an dem Verbindungsabschnitt (26) abstützt.

30. Fixationsvorrichtung nach einem der Ansprüche 19 bis 29, **dadurch gekennzeichnet, daß** das zweite Klemmstück (36) und das zweite Trägerelement (32) einstückig ausgebildet sind.

31. Fixationsvorrichtung nach einem der Ansprüche 19 bis 29, **dadurch gekennzeichnet, daß** das zweite Klemmstück (36) und das zweite Trägerelement zweiteilig ausgebildet sind.

32. Fixationsvorrichtung nach einem der Ansprüche 8 bis 31, **dadurch gekennzeichnet, daß** das zweite Trägerelement (32) in der Justierstellung um eine zweite Drehachse (30) drehbar am Verbindungsabschnitt (34) gelagert ist.

33. Fixationsvorrichtung nach Anspruch 32, **dadurch gekennzeichnet, daß** die erste (60) und die zweite Drehachse (30) parallel zueinander verlaufen.

34. Fixationsvorrichtung nach einem der Ansprüche 8 bis 33, **dadurch gekennzeichnet, daß** die Verbindungselementaufnahme (82) am ersten Trägerelement (46) um eine dritte Drehachse (80) verdrehbar gelagert ist und daß die dritte Drehachse (80) quer zu einer Längsachse (15) der Verbindungselementaufnahme (82) verläuft.

35. Fixationsvorrichtung nach Anspruch 34, **dadurch gekennzeichnet, daß** die dritte Drehachse (80) quer zur ersten und/oder zur zweiten Drehachse (60; 30) verläuft.

36. Fixationsvorrichtung nach einem der voranstehenden Ansprüche, **dadurch gekennzeichnet, daß** der Verbindungsabschnitt (26) das proximale Ende des Verankerungselements (12) bildet oder im Bereich des proximalen Endes des Verankerungselements (12) angeordnet ist.

37. Fixationsvorrichtung nach einem der Ansprüche 6 bis 36, **dadurch gekennzeichnet, daß** die Verbindungsabschnittaufnahme (34a) in Form einer Kugelgelenkaufnahme ausgebildet ist zum Aufnehmen eines kugeligen Verbindungsabschnitts (26).

38. Fixationsvorrichtung nach einem der voranstehenden Ansprüche, **dadurch gekennzeichnet, daß** das Spannglied (50; 122; 166; 172) eine Werkzeugaufnahme (70) aufweist zum Aufnehmen eines Spannwerkzeugs (72) und daß mit dem Spannwerkzeug (72) das Spannglied (50; 122; 166; 172) bewegbar ist.

39. Orthopädisches Fixationssystem (10) umfassend mindestens zwei in oder an einem Knochen (18) verankerbare Verankerungselemente (12), mindestens ein Verbindungselement (14) zum Verbinden der mindestens zwei Verankerungselemente (12) und mindestens eine orthopädische Fixationsvorrichtung (16; 120; 140; 150; 160; 170; 180; 190) zum Verbinden des mindestens einen Verbindungselements (14) mit einem Verbindungsabschnitt (26) eines der mindestens zwei Verankerungselemente (12), **dadurch gekennzeichnet, daß** die mindestens eine orthopädische Fixationsvorrichtung (16; 120; 140; 150; 160; 170; 180; 190) eine Fixationsvorrichtung (16; 120; 140; 150; 160; 170; 180; 190) nach einem der voranstehenden Ansprüche ist.

40. Fixationssystem nach Anspruch 39, **dadurch gekennzeichnet, daß** der Verbindungsabschnitt (26) gewindefrei und zylindrisch oder im wesentlichen zylindrisch geformt ist.

41. Fixationssystem nach Anspruch 39, **dadurch gekennzeichnet, daß** der Verbindungsabschnitt des Verankerungselements (12) kalottenförmig oder im wesentlichen kalottenförmig ist.

42. Fixationssystem nach einem der Ansprüche 39 bis 41, **dadurch gekennzeichnet, daß** das Verankerungselement (12) einen sein distales Ende bildenden Schraubengewindeabschnitt (20) zum Einschrauben in einen Knochen (18) umfaßt.

43. Fixationssystem nach einem der Ansprüche 39 bis 41, **dadurch gekennzeichnet, daß** das Verankerungselement (12) einen sein distales Ende bildenden Haken zum Verankern des Verankerungselements (12) in einem Knochen (18) umfaßt.

44. Fixationssystem nach einem der Ansprüche 39 bis 43, **dadurch gekennzeichnet, daß** das Verbindungselement (14) ein Stab oder eine Verbindungsplatte mit mindestens einem stabförmigen Plattenabschnitt ist.

## Claims

1. Orthopaedic fixation device (16; 120; 140; 150; 160; 170; 180; 190) for connecting a first anchoring element (12) having a connecting section (26) and being adapted to be anchored in or on a bone (18) to a connecting element (14) adapted to be connected to a second anchoring element adapted to be anchored in or on a bone, wherein the fixation device (16; 120; 140; 150; 160; 170; 180; 190) is movable relative to the connecting element (14) and to the anchoring element (12) in an adjusting position and is adapted to be secured on the connecting element (14) and on the anchoring element (12) in a fixing position, wherein a single tensioning member (50; 122; 166; 172) supported so as to be movable is provided for transferring the fixation device (16; 120; 140; 150; 160; 170; 180; 190) from the adjusting position and into the fixing position and vice versa, **characterized in that** a first clamping device is provided for clampingly securing the fixation device (16; 120; 140; 150; 160; 170; 180; 190) on the connecting element (14) in the fixing position, that a second clamping device is provided for clampingly securing the fixation device (16; 120; 140; 150; 160; 170; 180; 190) on the anchoring element (12) in the fixing position and that the first clamping device comprises the tensioning member (50; 122; 166; 172) and that the second clamping device comprises the tensioning member (50; 122; 166; 172).

2. Fixation device as defined in claim 1, **characterized in that** the fixation device (16; 120; 140; 150; 160; 170; 180; 190) comprises at least one first supporting element (46) and that the tensioning member (50; 122; 166; 172) is supported on the at least one first supporting element (46) so as to be rotatable.

3. Fixation device as defined in one of the preceding claims, **characterized in that** the fixation device (16; 120; 140; 150; 160; 170; 180; 190) comprises at least one first supporting element (46) and that the tensioning member (50; 122; 166; 172) is supported relative to the first supporting element (46) so as to be translatable.

4. Fixation device as defined in any one of the preceding claims, **characterized in that** in the adjusting position the connecting element (14) is displaceable relative to the anchoring element (12) parallel to itself towards or away from the anchoring element (12).

5. Fixation device as defined in any one of the preceding claims, **characterized in that** the fixation device (16; 120; 140; 150; 160; 170; 180; 190) has a connecting element receiving means (82) for accommodating the connecting element (14), said receiving means corresponding to the connecting element (14).

6. Fixation device as defined in any one of the preceding claims, **characterized in that** the fixation device (16; 120; 140; 150; 160; 170; 180; 190) has a connecting section receiving means (34) for accommodating the connecting section (26), said receiving means corresponding to the connecting section (26).

7. Fixation device as defined in claims 5 and 6, **characterized in that** the connecting section receiving means (34) is rotatable about a first axis of rotation (60) relative to the connecting element receiving means (82).

8. Fixation device as defined in claims 5 and 6 or as defined in claim 7, **characterized in that** the fixation device (16; 120; 140; 150; 160; 170; 180; 190) comprises a first supporting element (46) supporting the connecting element receiving means (82) and a second supporting element (32) supporting the connecting section receiving means (34) and being supported on the first supporting element (46) so as to be rotatable about a first axis of rotation (60) in the adjusting position.

9. Fixation device as defined in claims 7 or 8, **characterized in that** the tensioning member (50; 122; 166; 172) forms a bearing shaft defining the first axis of rotation (60).

10. Fixation device as defined in claims 8 or 9, **characterized in that** the first clamping device comprises a first clamping element (94; 126) having a first slide-on surface (96) abutting directly or indirectly on a first actuating surface of a first clamping member (84) of the first supporting element (46) and that as a result of movement of the tensioning member (50; 122; 166; 172) the first clamping element (94; 126) is movable in such a manner that the first slide-on surface (96) is able to slide on the first actuating surface and reduce a cross section of the connecting element receiving means (82) in such a manner that the connecting element (14) is adapted to be secured in the connecting element receiving means (82).

11. Fixation device as defined in claim 10, **characterized in that** a first force transfer member (86) arranged between the first slide-on surface (96) and the first actuating surface is provided for transferring a force from the first clamping element (94; 126) to the first clamping member (84).

12. Fixation device as defined in claim 10 or 11, **characterized in that** the first slide-on surface (96) and the first actuating surface (88) are inclined relative to the first axis of rotation (60).

13. Fixation device as defined in claim 10 or 11, **characterized in that** the first slide-on surface is a first outer surface of a first eccentric (126) rotatable about the first axis of rotation (60) and that the first actuating surface (88a) is curved concavely in the direction towards the first axis of rotation (60) at least in sections.

14. Fixation device as defined in any one of claims 10 to 13, **characterized in that** the first clamping element (94) is designed in the shape of a cone.

15. Fixation device as defined in any one of claims 10 to 14, **characterized in that** the first clamping element and the tensioning member (50; 122; 166; 172) are designed in one piece.

16. Fixation device as defined in any one of claims 10 to 14, **characterized in that** the first clamping element (94; 126) and the tensioning member (50; 122; 166; 172) are designed in two parts.

17. Fixation device as defined in claim 16, **characterized in that** the first clamping element and the tensioning member (50; 122, 166; 172) are connected to one another so as to be non-rotatable.

18. Fixation device as defined in any one of claims 10 to 17, **characterized in that** the tensioning member (50; 166) has a first external thread section (68), that the first clamping element (94) has a first internal thread section (98) corresponding to the first external thread section (68) and that the first clamping element (94) is displaceable parallel to itself as a result of rotation of the tensioning member (50; 166) about its longitudinal axis.

19. Fixation device as defined in any one of claims 8 to 18, **characterized in that** the second clamping device comprises a second clamping element (102; 124; 162; 174) having a second slide-on surface (104; 164) abutting directly or indirectly on a second actuating surface (44; 128) of the second supporting element (32), and that as a result of movement of the tensioning member (50; 122; 166; 172) the second clamping element (102; 124; 162; 174) is movable in such a manner that the second slide-on surface (104; 164) slides on the second actuating surface (44; 128) and a second clamping member (36; 152, 154) limiting the connecting section receiving means in sections is movable in such a manner that a cross section of the connecting section receiving means (34) is able to be reduced such that the connecting section (26) of the anchoring element (12) is adapted to be secured in the connecting section receiving means (34).

20. Fixation device as defined in claim 19, **characterized in that** the second slide-on surface (104; 164) and the second actuating surface (44) are inclined relative to the first axis of rotation (60).

21. Fixation device as defined in one of claims 19 or 20, **characterized in that** the second slide-on surface is a second outer surface of a second eccentric (124) rotatable about the first axis of rotation (60) and that the second actuating surface (128) is curved concavely in the direction towards the first axis of rotation (60) at least in sections.

22. Fixation device as defined in one of claims 19 or 20, **characterized in that** the second clamping element (102; 162; 174) is designed at least partially in the shape of a cone.

23. Fixation device as defined in any one of claims 19 to 22, **characterized in that** the second clamping element (166; 172) and the tensioning member (162; 174) are designed in one piece.

24. Fixation device as defined in any one of claims 19 to 22, **characterized in that** the second clamping element (102; 162) and the tensioning member (50; 166) are designed in two parts.

25. Fixation device as defined in claim 24, **characterized in that** the second clamping element (162; 174) and the tensioning member (50; 166) are connected to one another so as to be non-rotatable.

26. Fixation device as defined in any one of claims 17 to 25, **characterized in that** the tensioning member (50) has a second external thread section (64), that the second clamping element (102) has a second internal thread section (106) corresponding to the second external thread section (64) and that the second clamping element (102) is displaceable parallel to itself as a result of rotation of the tensioning member (50) about its longitudinal axis (60).

27. Fixation device as defined in claim 26, **characterized in that** the first and the second external thread sections (64, 68) are thread sections running in opposite directions.

28. Fixation device as defined in claim 27, **characterized in that** the first external thread section is a right-hand thread section (64) and that the second external thread section is a left-hand thread section (68).

29. Fixation device as defined in any one of claims 19 to 28, **characterized in that** in the fixing position the second supporting element (32) is supported on the connecting section (26) via the second clamping member (36).

30. Fixation device as defined in any one of claims 19 to 29, **characterized in that** the second clamping member (36) and the second supporting element (32) are designed in one piece.

31. Fixation device as defined in any one of claims 20 to 30, **characterized in that** the second clamping member (36) and the second supporting element are designed in two parts.

32. Fixation device as defined in any one of claims 8 to 31, **characterized in that** the second supporting element (32) is supported on the connecting section (34) so as to be rotatable about a second axis of rotation (30) in the adjusting position.

33. Fixation device as defined in claim 32, **characterized in that** the first (60) and the second axes of rotation (30) extend parallel to one another.

34. Fixation device as defined in any one of claims 8 to 33, **characterized in that** the connecting element receiving means (82) is supported on the first supporting element (46) so as to be rotatable about a third axis of rotation (80) and that the third axis of rotation (80) extends transversely to a longitudinal axis (15) of the connecting element receiving means (82).

35. Fixation device as defined in claim 34, **characterized in that** the third axis of rotation (80) extends transversely to the first and/or to the second axis of rotation (60; 30).

36. Fixation device as defined in any one of the preceding claims, **characterized in that** the connecting section (26) forms the proximal end of the anchoring element (12) or is arranged in the area of the proximal end of the anchoring element (12).

37. Fixation device as defined in any one of claims 6 to 36, **characterized in that** the connecting section receiving means (34a) is designed in the form of a spherical joint receiving means for accommodating a spherical connecting section (26).

38. Fixation device as defined in any one of the preceding claims, **characterized in that** the tensioning member (50; 122; 166; 172) has a tool receiving means (70) for accommodating a tensioning tool (72) and that the tensioning member (50; 122; 166; 172) is movable with the tensioning tool (72).

39. Orthopaedic fixation system (10) comprising at least two anchoring elements (12) adapted to be anchored in or on a bone (18), at least one connecting element (14) for connecting the at least two anchoring elements (12) and at least one orthopaedic fixation device (16; 120; 140; 150; 160; 170; 180; 190) for connecting the at least one connecting element (14) to a connecting section (26) of one of the at least two anchoring elements (12), **characterized in that** the at least one orthopaedic fixation device (16; 120; 140; 150; 160; 170; 180; 190) is a fixation device (16; 120; 140; 150; 160; 170; 180; 190) as defined in any one of the preceding claims.

40. Fixation system as defined in claim 39, **characterized in that** the connecting section (26) is designed so as to be thread-free and is cylindrical or essentially cylindrical in shape.

41. Fixation system as defined in claim 39, **characterized in that** the connecting section of the anchoring element (12) is in the shape of a spherical cap or essentially in the shape of a spherical cap.

42. Fixation system as defined in any one of claims 39 to 41, **characterized in that** the anchoring element (12) comprises a screw thread section (20) forming its distal end for screwing into a bone (18).

43. Fixation system as defined in any one of claims 39 to 41, **characterized in that** the anchoring element (12) comprises a hook forming its distal end for anchoring the anchoring element (12) in a bone (18).

44. Fixation system as defined in any one of claims 39 to 43, **characterized in that** the connecting element (14) is a bar or a connecting plate with at least one bar-like plate section.

## Revendications

1. Dispositif de fixation orthopédique (16; 120; 140; 150; 160; 170; 180; 190) pour relier un premier élément d'ancrage (12), qui peut être ancré dans ou à un os (18) et présente un tronçon de liaison (26), à un élément de liaison (14), qui peut être relié à un deuxième élément d'ancrage pouvant être ancré dans ou à un os, le dispositif de fixation (16; 120; 140; 150; 160; 170; 180; 190) pouvant être déplacé par rapport à l'élément de liaison (14) et à l'élément d'ancrage (12) dans une position d'ajustage, et être fixé à l'élément de liaison (14) et à l'élément d'ancrage (12) dans une position de fixation, un seul et unique organe de serrage (50; 122; 166; 172) monté mobile étant prévu pour transférer le dispositif de fixation (16; 120; 140; 150; 160; 170; 180; 190) de la position d'ajustage et vers la position de fixation et inversement,
**caractérisé en ce qu'**il est prévu un premier dispositif de blocage pour fixer de manière bloquée le dispositif de fixation (16; 120; 140; 150; 160; 170; 180; 190) à l'élément de liaison (14) dans la position de fixation, **en ce qu'**il est prévu un deuxième dispositif de blocage pour fixer de manière bloquée le dispositif de fixation (16; 120; 140; 150; 160; 170; 180; 190) à l'élément d'ancrage (12) dans la position de fixation, et **en ce que** le premier dispositif de blocage comprend l'organe de serrage (50; 122; 166; 172) et **en ce que** le deuxième dispositif de blocage comprend l'organe de serrage (50; 122; 166; 172).

2. Dispositif de fixation selon la revendication 1, **caractérisé en ce que** le dispositif de fixation (16; 120; 140; 150; 160; 170; 180; 190) comprend au moins un premier élément de support (46) et **en ce que** l'organe de serrage (50; 122; 166; 172) est monté de manière rotative sur ledit au moins un élément de support (46).

3. Dispositif de fixation selon l'une des revendications précédentes, **caractérisé en ce que** le dispositif de fixation (16; 120; 140; 150; 160; 170; 180; 190) comprend au moins un premier élément de support (46) et **en ce que** l'organe de serrage (50; 122; 166; 172) est monté de manière translatable par rapport au premier élément de support (46).

4. Dispositif de fixation selon l'une des revendications précédentes, **caractérisé en ce que** dans la position d'ajustage, l'élément de liaison (14) peut coulisser, par rapport à l'élément d'ancrage (12), parallèlement à lui-même en direction de l'élément d'ancrage (12) ou de manière à s'éloigner de celui-ci.

5. Dispositif de fixation selon l'une des revendications précédentes, **caractérisé en ce que** le dispositif de fixation (16; 120; 140; 150; 160; 170; 180; 190) comporte un logement d'accueil d'élément de liaison (82) correspondant à l'élément de liaison (14) et destiné à accueillir l'élément de liaison (14).

6. Dispositif de fixation selon l'une des revendications précédentes, **caractérisé en ce que** le dispositif de fixation (16; 120; 140; 150; 160; 170; 180; 190) comporte un logement d'accueil de tronçon de liaison (34) correspondant au tronçon de liaison (26) et destiné à accueillir le tronçon de liaison (26).

7. Dispositif de fixation selon la revendication 5 et la revendication 6, **caractérisé en ce que** le logement d'accueil de tronçon de liaison (34) peut tourner par rapport au logement d'accueil d'élément de liaison (82), autour d'un premier axe de rotation (60).

8. Dispositif de fixation selon la revendication 5 et la revendication 6 ou selon la revendication 7, **caractérisé en ce que** le dispositif de fixation (16; 120; 140; 150; 160; 170; 180; 190) comprend un premier élément de support (46) portant le logement d'accueil d'élément de liaison (82), et un deuxième élément de support (32), qui porte le logement d'accueil de tronçon de liaison (34), et est monté sur le premier élément de support (46) de manière à pouvoir tourner autour d'un premier axe de rotation (60) dans la position d'ajustage.

9. Dispositif de fixation selon la revendication 7 ou la revendication 8, **caractérisé en ce que** l'organe de serrage (50; 122; 166; 172) forme un axe de palier définissant le premier axe de rotation (60).

10. Dispositif de fixation selon l'une des revendications 8 ou 9, **caractérisé en ce que** le premier dispositif de blocage comprend un premier élément de blocage (94; 126), qui présente une première surface de glissement (96) s'appuyant directement ou indirectement sur une première surface d'actionnement d'une première pièce de blocage (84) du premier élément de support (46), et **en ce que** suite à un mouvement de l'organe de serrage (50; 122; 166; 172) le premier élément de blocage (94; 126) peut être déplacé de manière telle, que la première surface de glissement (96) puisse coulisser sur la première surface d'actionnement et conduise à un rétrécissement d'une section transversale du logement d'accueil d'élément de liaison (82) de façon à pouvoir fixer et immobiliser l'élément de liaison (14) dans le logement d'accueil d'élément de liaison (82).

11. Dispositif de fixation selon la revendication 10, **caractérisé en ce qu'**il est prévu un premier organe de transmission de force (86), qui est agencé entre la première surface de glissement (96) et la première surface d'actionnement pour la transmission d'une force du premier élément de blocage (94; 126) à la première pièce de blocage (84).

12. Dispositif de fixation selon la revendication 10 ou la revendication 11, **caractérisé en ce que** la première surface de glissement (96) et la première surface d'actionnement (88) sont inclinées par rapport au premier axe de rotation (60).

13. Dispositif de fixation selon la revendication 10 ou la revendication 11, **caractérisé en ce que** la première surface de glissement est une première surface extérieure d'un premier excentrique (126) pouvant tourner autour du premier axe de rotation (60), et **en ce que** la première surface d'actionnement (88a) est courbée de manière concave, au moins par secteurs, en direction du premier axe de rotation (60).

14. Dispositif de fixation selon l'une des revendications 10 à 13, **caractérisé en ce que** le premier élément de blocage (94) est réalisé sous la forme d'un cône.

15. Dispositif de fixation selon l'une des revendications 10 à 14, **caractérisé en ce que** le premier élément de blocage et l'organe de serrage (50; 122; 166; 172) sont réalisés d'un seul tenant en une seule pièce.

16. Dispositif de fixation selon l'une des revendications 10 à 14, **caractérisé en ce que** le premier élément de blocage (94; 126) et l'organe de serrage (50; 122; 166; 172) sont réalisés en deux pièces.

17. Dispositif de fixation selon la revendication 16, **caractérisé en ce que** le premier élément de blocage et l'organe de serrage (50; 122; 166; 172) sont reliés de manière fixe en rotation.

18. Dispositif de fixation selon l'une des revendications 10 à 17, **caractérisé en ce que** l'organe de serrage (50; 166) présente un premier tronçon de filetage extérieur (68), **en ce que** le premier élément de blocage (94) présente un premier tronçon de filetage intérieur (98) correspondant au premier tronçon de filetage extérieur (68), et **en ce que** par rotation de l'organe de serrage (50; 166) autour de son axe longitudinal, le premier élément de blocage (94) peut coulisser parallèlement à lui-même.

19. Dispositif de fixation selon l'une des revendications 8 à 18, **caractérisé en ce que** le deuxième dispositif de blocage comprend un deuxième élément de blocage (102; 124; 162; 174), qui présente une deuxième surface de glissement (104; 164) s'appuyant directement ou indirectement sur une deuxième surface d'actionnement (44; 128) du deuxième élément de support (32), et **en ce que** suite à un mouvement de l'organe de serrage (50; 122; 166; 172) le deuxième élément de blocage (102; 124; 162; 174) peut être déplacé de manière telle, que la deuxième surface de glissement (104; 164) puisse coulisser sur la deuxième surface d'actionnement (44; 28) et qu'une deuxième pièce de blocage (36 ; 152, 154) délimitant par tronçons le logement d'accueil de tronçon de liaison puisse être déplacée de façon à permettre le rétrécissement d'une section transversale du logement d'accueil de tronçon de liaison (34) de façon à pouvoir fixer et immobiliser le tronçon de liaison (26) de l'élément d'ancrage (12) dans le logement d'accueil de tronçon de liaison (34).

20. Dispositif de fixation selon la revendication 19, **caractérisé en ce que** la deuxième surface de glissement (104; 164) et la deuxième surface d'actionnement (44) sont inclinées par rapport au premier axe de rotation (60).

21. Dispositif de fixation selon l'une des revendications 19 ou 20, **caractérisé en ce que** la deuxième surface de glissement est une deuxième surface extérieure d'un deuxième excentrique (124) pouvant tourner autour du premier axe de rotation (60), et **en ce que** la deuxième surface d'actionnement (128) est courbée de manière concave, au moins par secteurs, en direction du premier axe de rotation (60).

22. Dispositif de fixation selon l'une des revendications 19 ou 20, **caractérisé en ce que** le deuxième élément de blocage (102; 162; 174) est réalisé sous la forme d'un cône.

23. Dispositif de fixation selon l'une des revendications 19 à 22, **caractérisé en ce que** le deuxième élément de blocage (166 ; 172) et l'organe de serrage (162; 174) sont réalisés d'un seul tenant en une seule pièce.

24. Dispositif de fixation selon l'une des revendications 19 à 22, **caractérisé en ce que** le deuxième élément de blocage (102; 162) et l'organe de serrage (50; 166) sont réalisés en deux pièces.

25. Dispositif de fixation selon la revendication 24, **caractérisé en ce que** le deuxième élément de blocage (162; 174) et l'organe de serrage (50; 166) sont reliés de manière fixe en rotation.

26. Dispositif de fixation selon l'une des revendications 17 à 25, **caractérisé en ce que** l'organe de serrage (50) présente un deuxième tronçon de filetage extérieur (64), **en ce que** le deuxième élément de blocage (102) présente un deuxième tronçon de filetage intérieur (106) correspondant au deuxième tronçon de filetage extérieur (64), et **en ce que** par rotation de l'organe de serrage (50) autour de son axe longitudinal (60), le deuxième élément de blocage (102) peut coulisser parallèlement à lui-même.

27. Dispositif de fixation selon la revendication 26, **caractérisé en ce que** le premier et le deuxième tronçon de filetage extérieur (64, 68) sont des tronçons de filetage de pas opposés.

28. Dispositif de fixation selon la revendication 27, **caractérisé en ce que** le premier tronçon de filetage extérieur est un tronçon de filetage à droite (64) et le deuxième tronçon de filetage extérieur et un tronçon de filetage à gauche (68).

29. Dispositif de fixation selon l'une des revendications 19 à 28, **caractérisé en ce que** dans la position de fixation, le deuxième élément de support (32) s'appuie par l'intermédiaire de la deuxième pièce de blocage (36), sur le tronçon de fixation (26).

30. Dispositif de fixation selon l'une des revendications 19 à 29, **caractérisé en ce que** la deuxième pièce de blocage (36) et le deuxième élément de support (32) sont réalisés d'un seul tenant en une pièce.

31. Dispositif de fixation selon l'une des revendications 19 à 29, **caractérisé en ce que** la deuxième pièce de blocage (36) et le deuxième élément de support sont réalisés en deux pièces.

32. Dispositif de fixation selon l'une des revendications 8 à 31, **caractérisé en ce que** dans la position d'ajustage, le deuxième élément de support (32) est monté sur le tronçon de liaison (34) de manière rotative autour d'un deuxième axe de rotation (30).

33. Dispositif de fixation selon la revendication 32, **caractérisé en ce que** le premier (60) et le deuxième axe de rotation (30) s'étendent parallèlement l'un à l'autre.

34. Dispositif de fixation selon l'une des revendications 8 à 33, **caractérisé en ce que** le logement d'accueil d'élément de liaison (82) est monté sur le premier élément de support (46) de manière rotative autour d'un troisième axe de rotation (80), et **en ce que** le troisième axe de rotation (80) s'étend transversalement à un axe longitudinal (15) du logement d'accueil d'élément de liaison (82).

35. Dispositif de fixation selon la revendication 34, **caractérisé en ce que** le troisième axe de rotation (80) s'étend transversalement au premier et/ou au deuxième axe de rotation (60; 30).

36. Dispositif de fixation selon l'une des revendications précédentes, **caractérisé en ce que** le tronçon de liaison (26) forme l'extrémité proximale de l'élément d'ancrage (12) ou est agencé dans la zone de l'extrémité proximale de l'élément d'ancrage (12).

37. Dispositif de fixation selon l'une des revendications 6 à 36, **caractérisé en ce que** le logement d'accueil de tronçon de liaison (34a) est réalisé sous la forme d'un logement d'articulation à rotule sphérique pour accueillir un tronçon de liaison (26) en forme de rotule sphérique.

38. Dispositif de fixation selon l'une des revendications précédentes, **caractérisé en ce que** l'organe de serrage (50; 122; 166; 172) présente un logement d'accueil d'outil (70) destiné à accueillir un outil de serrage (72), et **en ce que** l'outil de serrage (72) permet de déplacer l'organe de serrage (50; 122; 166; 172).

39. Système de fixation orthopédique (10) comprenant au moins deux éléments d'ancrage (12) pouvant être ancrés dans ou sur un os (18), au moins un élément de liaison (14) pour relier lesdits au moins deux éléments d'ancrage (12), et au moins un dispositif de fixation orthopédique (16; 120; 140; 150; 160; 170; 180; 190) pour relier ledit au moins élément de liaison (14) avec un élément de liaison (26) de l'un desdits au moins deux éléments d'ancrage (12), **caractérisé en ce que** ledit au moins un dispositif de fixation orthopédique (16; 120; 140; 150; 160; 170; 180; 190) est un dispositif de fixation (16; 120; 140; 150; 160; 170; 180; 190) selon l'une des revendications précédentes.

40. Système de fixation selon la revendication 39, **caractérisé en ce que** le tronçon de liaison (26) est sans filetage et cylindrique ou sensiblement de forme cylindrique.

41. Système de fixation selon la revendication 39, **caractérisé en ce que** le tronçon de liaison de l'élément d'ancrage (12) présente une forme de calotte ou est sensiblement en forme de calotte.

42. Système de fixation selon l'une des revendications 39 à 41, **caractérisé en ce que** l'élément d'ancrage (12) comprend un tronçon de filetage de vis (20) formant son extrémité distale et destiné à être vissé dans un os (18).

43. Système de fixation selon l'une des revendications 39 à 41, **caractérisé en ce que** l'élément d'ancrage (12) comprend un crochet formant son extrémité distale, destiné à ancrer l'élément d'ancrage (12) dans un os (18).

44. Système de fixation selon l'une des revendications 39 à 43, **caractérisé en ce que** l'élément de liaison (14) est une tige ou une plaque de liaison avec au moins un tronçon de plaque en forme de tige.
